# EUROPEAN PATENT APPLICATION

(11) **EP 3 939 659 A1**
(43) Date of publication of application: **19.01.2022**
(21) Application number: 20771088.0
(22) Date of filing: 12.03.2020
(51) Int. Cl.: A61P 9/10, A61P 13/12, A61P 25/00, A61P 25/02, A61P 27/02, A61K 31/407, A61K 31/502

(54) **DRUG AND METHOD FOR TREATING OR PREVENTING COMPLICATIONS FROM DIABETES, USING SAID DRUG**

(30) Priority: 12.03.2019 JP 2019044670
(71) Applicant: Showa University, Tokyo 142-8555 (JP); TMS Co., Ltd., Fuchi-shi, Tokyo 183-0023 (JP)
(72) Inventor: SHIBATA, Keita, Tokyo 142-8555 (JP); SHINOUCHI, Ryosuke, Tokyo 142-8555 (JP); HASHIMOTO, Terumasa, Tokyo 142-8555 (JP); NOBE, Koji, Tokyo 142-8555 (JP)
(74) Representative: Beck Greener LLP
(86) International application number: PCT/JP2020/010967
(87) International publication number: WO 2020/184691

(57) **Abstract**

A drug containing a compound represented by formula (I) as an active ingredient, used for complications from diabetes.

## Description

### Technical Field

The present disclosure relates to a drug and a method for treating or preventing diabetes complications using this drug.

### Background Art

Insulin, which is a hormone produced by pancreatic β cells, promotes uptake of glucose in the blood, and the like, by cells such as in the muscles, liver, and fat when glucose concentration in the blood (hereinafter also referred to as "blood glucose level") is elevated to adjust the blood glucose level in the blood to a fixed concentration. Diabetes is a condition in which the mechanism of lowering the blood glucose level causes problems due to a lack of insulin secretion in the body or due to a decrease in cell sensitivity to insulin.

When the blood glucose level in the blood continues to be high (also called "high blood glucose state"), damage to small blood vessels in the kidneys, retina, nerves, and the like or arteriosclerosis and the like are further promoted, and major vascular diseases such as myocardial infarction, cerebral infarction, and obstructive arteriosclerosis of the legs are known (so-called complications of diabetes) to begin onset.

Treatment of diabetes is generally conducted mainly by lowering the blood glucose level in the blood, and for example, hypoglycemic agents such as an insulin injection or an insulin secretion promoter, an insulin resistance improving agent, and an α-glucosidase inhibitor are widely applied as treatment methods.

On the other hand, SMTP (*Stachybotrys microspora* triprenyl phenol) compounds are a group of compounds having a triprenylphenol skeleton produced by filamentous fungi, and according to JP 2004-224737 A, JP 2004-224738 A, and WO 2007/111203, they are known to have thrombolytic and antiangiogenic effects. Concerning thrombolysis-promoting action, according to FEBS Letter 1997; 418:58-62, SMTP compounds lead to a conformational change of plasminogen. As a result, it is suggested that the mechanism of action of SMTP compounds is that the sensitivity of plasminogen to t-PA and the binding of plasminogen to thromboses and the like are increased to promote the dissolution of thromboses. Furthermore, according to J Biol Chem 2014; 289:35826-35838, SMTP compounds have also shown excellent anti-inflammatory effects.

### Summary of Invention

### Problem to Be Solved by Invention

It is known that blood vessel injuries occur due to continued high blood glucose, causing various complications resulting from a high blood glucose state such as diabetic neuropathy, diabetic kidney disease (DKD), and diabetic retinopathy. Diabetic nephropathy (DN) is one example of diabetic kidney disease. Even among diabetes complications, for example, diabetic neuropathy is highly likely to develop and develops from an early stage of the onset of diabetes, presenting a variety of clinical features. Additionally, advanced diabetic neuropathy reduces the quality of life (QoL) of the patient and also affects vital prognosis, as shown by high mortality and the like in patients with autonomic nervous disorders. Furthermore, for example, diabetic kidney disease requires treatment such as dialysis therapy or kidney transplantation when the symptoms progress. Thus, further development of drugs and treatment methods or prevention methods for diabetes complications is required.

JP 2004-224737 A, JP 2004-224738 A, WO 2007-040082, WO 2007/111203, FEBS Letter 1997; 418:58-62, and J Biol Chem 2014; 289:35826-35838 do not describe or suggest any details of the effects of the compounds represented by formula (I) on diabetes complications.

Upon examination, the present inventors found that the compound represented by formula (I) has effects in the treatment or prevention of diabetes complications such as diabetic neuropathy or diabetic kidney disease.

Diabetic neuropathy is one of the most common diabetes complications. The details of the mechanism of onset thereof are unknown, but it is suggested that accumulation of sorbitol in the body, deficiency of nutrient substances, blood flow disorders associated therewith, and the like due to a high blood glucose state may also be factors. The reason the foregoing effect is obtained is unknown, but the inventors postulate that the compound represented by formula (I) is excellent in the treatment or prevention of diabetes complications such as diabetic neuropathy due to improving blood flow disorders.

Diabetic kidney disease is also one of the most common diabetes complications. The details of the mechanism of onset thereof are unknown, but it is suggested that a high blood glucose state damages renal tubules and breaks small blood vessels in the glomeruli of the kidney, making it impossible to filter waste products The reason the foregoing effect is obtained is unknown, but the inventors postulate that the compound represented by formula (I) is excellent in the treatment or prevention of diabetes complications such as diabetic kidney disease due to improving blood vessel disorders.

A problem to be solved by embodiments of the present disclosure is to provide a drug excellent in therapeutic or prophylactic effect on diabetes complications such as diabetic neuropathy or diabetic kidney disease and a new use of a compound represented by formula (I) as a medicine.

### Means for Solving Problem

Means for solving the foregoing problem include the following aspects.
<1> A drug used for diabetes complications, including a compound represented by formula (I) below as an active ingredient: 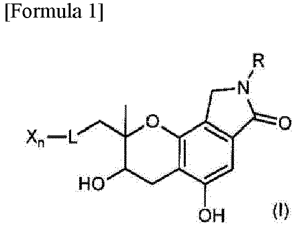 wherein, in formula (I), L represents a 4 to 10 carbon aliphatic hydrocarbon group, X represents a hydroxy group or a carboxy group, n represents an integer of 0 to 2, and R represents a hydrogen atom or a substituent having a molecular weight of 1,000 or less.
<2> The drug according to <1>, wherein the compound represented by the formula (I) is a compound represented by formula (IA) below: 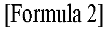 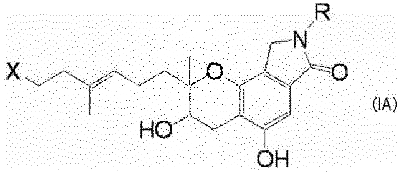 In formula (IA), X is -CHY-C(CH₃)₂Z, Y and Z are each independently -H or -OH or form a single bond together, and R represents a hydrogen atom or a substituent having a molecular weight of 1,000 or less.
<3> The drug according to <1> or <2>, wherein the compound represented by the formula (I) is a compound represented by formula (II) or formula (III) below: 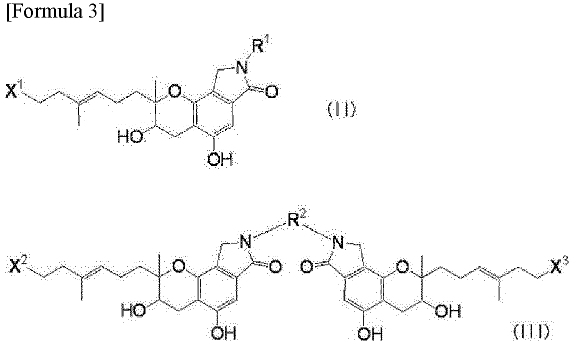 in formula (II) or formula (III), X¹, X², and X³ are each independently -CHY-C(CH₃)₂Z, Y and Z are each independently -H or -OH or form a single bond together, and R¹ represents any one of the following (A) to (D):
   (A) a residue obtained by removing a single amino group from an amino compound selected from the group consisting of natural amino acids, D isomers of natural amino acids, and compounds obtained by substituting at least one carboxyl group with a hydrogen atom, a hydroxy group, or a hydroxymethyl group in natural amino acids and D isomers of natural amino acids (however, this excludes -(CH)₂-OH)
   (B) an aromatic group having at least one selected from the group consisting of a carboxyl group, a hydroxyl group, a sulfonic acid group, and a secondary amino group as a substituent or as a part of a substituent, or an aromatic group containing a secondary amino group and optionally containing a nitrogen atom
   (C) an aromatic amino acid residue represented by formula (II-1) (in the formula, R³ are each independently and optionally a substituent, and if present, represent a hydroxy group, a carboxyl group, or a 1 to 5 carbon alkyl group, n represents an integer of 0 or 1, m represents an integer of 0 to 5, and ^{∗} represents a binding site) 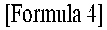 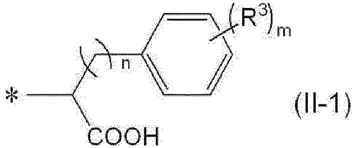
   (D) a substituent represented by -L¹-L²-R⁴- (in the formula, L¹ represents a linking group which is a 1 to 4 carbon alkene group having a carboxy group, L² represents a linking group represented by -NH-C(=O)- or -NH-C(=S)-NH-, and R⁴ is a 9-fluorenylalkoxy group having a 1 to 3 carbon alkoxy group or a polyheterocyclic group represented by formula (II-2) below (in the formula (II-2), ^{∗} represents a binding site): 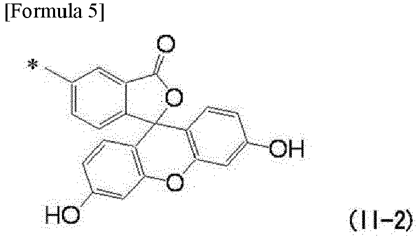 and R² represents a residue obtained by removing two amino groups from an amino compound selected from the groups consisting of: natural amino acids having two amino groups, D isomers of natural amino acids having two amino groups, compounds in which at least one carboxyl group in a natural amino acid having two amino groups or a D isomer of a natural amino acid having two amino groups is replaced with a hydrogen atom, a hydroxyl or a hydroxymethyl group, and compounds represented by H₂N-CH(COOH)-(CH₂)ₙ-NH₂ (n is an integer from 0 to 9) and H₂N-CH(COOH)-(CH₂)ₘ-Sₚ-(CH₂)_{q}-CH(COOH)-NH₂ (m, p and q are each independently integers from 0 to 9).
<4> The drug according any one of <1> to <3>, wherein the compound represented by formula (I) includes at least one selected from the group consisting of SMTP-0 below, SMTP-1 below, SMTP-4 below, SMTP-5D below, SMTP-6 below, SMTP-7 below, SMTP-8 below, SMTP-11 to 14 below, SMTP-18 to 29 below, SMTP-36 below, SMTP-37 below, SMTP-42 below, SMTP-43 below, SMTP-43D below, SMTP-44 below, SMTP-44D below, SMTP-46 below, and SMTP-47 below: 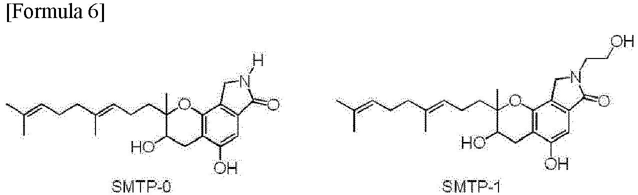 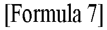 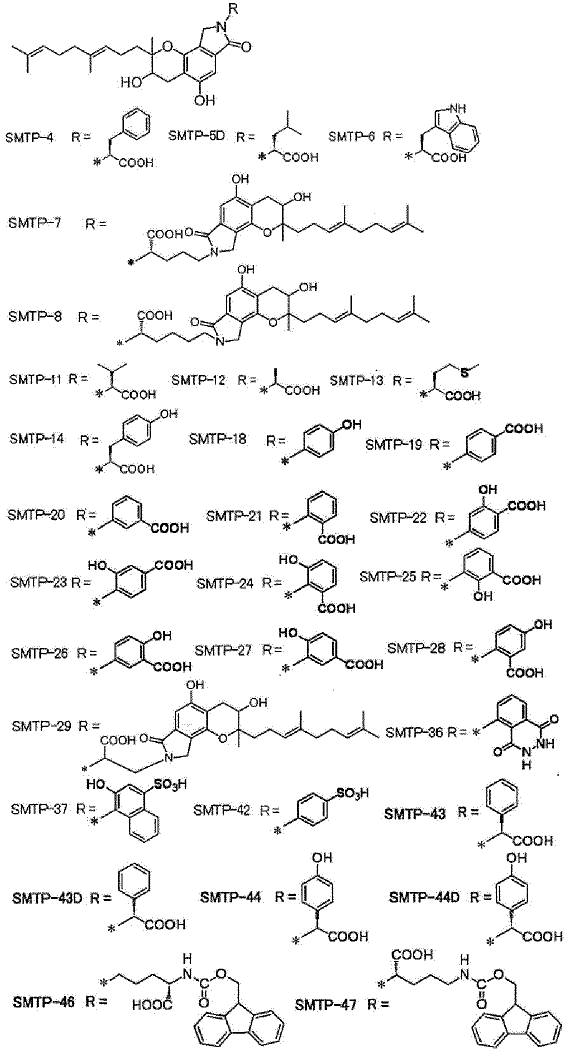 and in the formula, ^{∗} represents a binding site.
<5> The drug according to <4>, wherein the compound represented by formula (I) includes the SMTP-44D.
<6> The drug according to <4>, wherein the compound represented by formula (I) includes the SMTP-27.
<7> The drug according to any one of <1> to <6>, wherein the diabetes complication is diabetic neuropathy.
<8> The drug according to <7>, wherein the diabetic neuropathy is a polyneuropathy.
<9> The drug according to <7>, wherein the diabetic neuropathy is a mononeuropathy.
<10> The drug according to any one of <7> to <9>, wherein the diabetic neuropathy is neuropathy due to type 2 diabetes.
<11> The drug according to any one of <1> to <6>, wherein the diabetes complication is diabetic kidney disease.
<12> The drug according to <11>, wherein the diabetic kidney disease is diabetic nephropathy.
<13> The drug according to <12>, wherein the diabetic nephropathy is
   a disease satisfying any one condition selected from the group consisting of:
   eGFR value (mL/min/1.73 m²) being 30 or more and less than 90 and urinary albumin value (mg/gCr) being less than 30,
   eGFR value (mL/min/1.73 m²) being 30 or more and less than 90 and urinary albumin value (mg/gCr) being 30 or more, and
   eGFR value (mL/min/1.73 m²) being less than 30.
<14> The drug according to any one of <11> to <13>, wherein the diabetic kidney disease is nephropathy due to type 2 diabetes.
<15> A method for treating or preventing a diabetes complication in a subject having a diabetes complication or at risk of developing a diabetes complication, including administering a dose of the drug according to any one of <1> to <14> which is effective for treating or preventing diabetes complications to the subject.
<16> The method according to <15>, wherein the dose effective for treating or preventing the diabetes complication is 0.001 mg/kg of body weight to 200 mg/kg of body weight per dose to an adult.
<17> A compound of the foregoing formula (I) for treating or preventing diabetes complications.
<18> Use of a compound of the foregoing formula (I) in manufacturing a therapeutic drug or prophylactic drug for diabetes complications.

### Effect of Invention

According to an embodiment of the present disclosure, it is possible to provide a drug excellent in therapeutic or prophylactic effect on diabetes complications such as diabetic neuropathy or diabetic kidney disease and a new use of a compound represented by formula (I) as a medicine.

### Mode for Carrying Out Invention

The contents of the present disclosure will be described in detail below. The description of the constituent elements described below may be based on representative embodiments of the present disclosure, but the present disclosure is not limited to such embodiments.

In the numerical range described stepwise in the present disclosure, the upper limit value or the lower limit value described in one numerical range may be replaced with the upper limit value or the lower limit value of another numerical range described stepwise. Further, in the numerical range described in the present disclosure, the upper limit value or the lower limit value of the numerical range may be replaced with the values shown in the examples.

Furthermore, when there is a plurality of substances applicable to each component in the composition, the amount of each component in the composition such as a drug in the present disclosure means the total amount of the plurality of applicable substances present in the composition, unless otherwise specified.

In addition, in the notation of a group (group of atoms) in the present specification, notation that does not describe substitution or non-substitution includes those having no substituent as well as those having a substituent.

Moreover, the term "process" in the present specification not only includes independent processes but also includes processes that cannot be clearly distinguished from other processes as long as the intended purpose of the process is achieved. Also, in the present disclosure, "% by mass" and "% by weight" are synonymous, and "parts by mass" and "parts by weight" are synonymous.

Furthermore, in the present disclosure, a combination of two or more preferable embodiments is a further preferable embodiment.

Hereinafter, the present disclosure will be described in detail.

### (Drug)

The drug of the present disclosure is a drug used for diabetes complications which contains a compound represented by the above formula (I) as an active ingredient.

### <Compound Represented by Formula (I)>

The drug of the present disclosure contains a compound represented by formula (I). 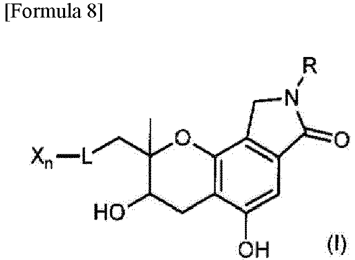 In formula (I), L represents a 4 to 10 carbon aliphatic hydrocarbon group, X represents a hydroxy group or a carboxy group, n represents an integer of 0 to 2, and R represents a hydrogen atom or a substituent having a molecular weight of 1,000 or less.

The 4 to 10 carbon aliphatic hydrocarbon group represented by L may be linear, branched, or cyclic. In addition, it may also contain an unsaturated bond. Among these, it is preferably an aliphatic hydrocarbon group that may contain a linear or branched unsaturated bond.

In formula (I), the group represented by -L-Xₙ is preferably represented by any of the following formula (V) and chemical formulas (Y1) to (Y4).

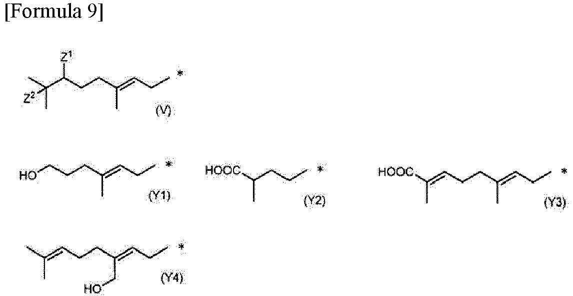

In the formula (V), Z¹ and Z² are each independently a hydrogen atom or a hydroxyl group, or they form a single bond together. Note that "^{∗}" in the chemical formula represents a binding site.

From the viewpoint of therapeutic or prophylactic effect on diabetes complications such as diabetic neuropathy or diabetic kidney disease, a substituent having a molecular weight of no less than 1 and no more than 800 is preferable as the substituent having a molecular weight of 1,000 or less in R in formula (I), a substituent having a molecular weight of no less than 15 and no more than 700 is more preferable, and a substituent having a molecular weight of no less than 15 and no more than 600 is further preferable.

Examples of R in formula (I) include α-amino acids (in this case, the nitrogen atom bonded to R corresponds to the α-amino group of the α-amino acid). The α-amino acid is not particularly limited and may be a natural amino acid or a non-natural amino acid. Further, it may be an amino acid derivative in which a substituent is introduced into a natural amino acid. Furthermore, when the α-amino acid has two or more amino groups, any amino group may be removed.

Among these, the α-amino acid is preferably a natural amino acid, a D isomer of a natural amino acid, or phenylalanine or phenylglycine that may have at least one substituent selected from the group consisting of a hydroxyl group, a carboxyl group, and a 1 to 5 carbon alkyl group, more preferably a natural amino acid, a D isomer of a natural amino acid, or phenylglycine that may have at least one substituent selected from the group consisting of a hydroxyl group, a carboxyl group, and a 1 to 5 carbon alkyl group.

Note that unless otherwise specified, "amino acid" in the present disclosure may be an L isomer amino acid or a D isomer amino acid.

The natural amino acid is not particularly limited as long as it is a naturally occurring amino acid. For example, glycine, alanine, threonine, valine, isoleucine, tyrosine, cysteine, cystine, methionine, histidine, aspartic acid, glutamic acid, asparagine, glutamine, arginine, lysine, hydroxylysine, ornithine, citrulline, homocysteine, 3,4-dihydroxyphenylalanine, homocystine, diaminopimelic acid, diaminopropionic acid, serine, leucine, phenylalanine, tryptophan, and the like are included.

Examples of the substituent in the amino acid derivative in which the substituent is introduced into the natural amino acid include a nitro group, a hydroxyl group, a 7 to 16 carbon arylalkyl group, a ureido group, a thioureido group, a carboxyl group, a group formed by removing one hydrogen atom from fluorescamine, and the like. Substituents in the amino acid derivative may further have a substituent if possible. The substituents contained in the substituents are the same as the substituents in the amino acid derivative.

Examples of R in formula (I) include amino sugars (in this case, the nitrogen atom bonded to R corresponds to the amino group of the amino sugar). The amino sugar is not particularly limited as long as it is a sugar derivative having at least one amino group. Specific examples thereof may include glucosamine, galactosamine, mannosamine, neuraminic acid, and the like.

Examples of R in formula (I) include an aromatic group having at least one selected from the group consisting of a carboxyl group, a hydroxyl group, a sulfonic acid group, and a secondary amino group as a substituent or as a part of a substituent and an aromatic group containing a secondary amino group and optionally containing a nitrogen atom as well. Examples of the aromatic group include a group represented by the following structural formula. In each structural formula, ^{∗} represents a binding site.

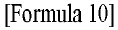

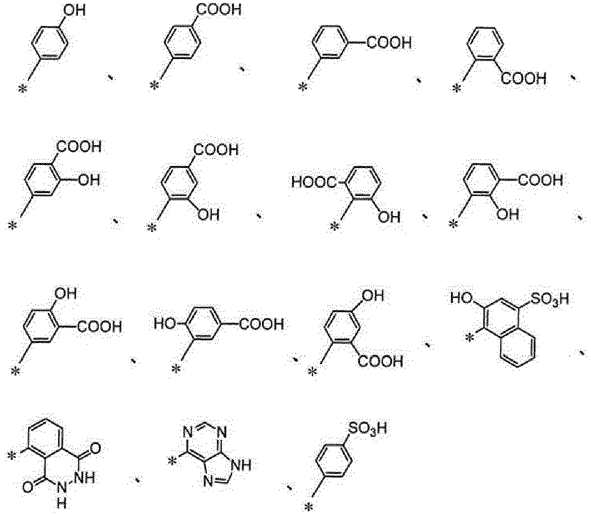

Examples of R in formula (I) include an aromatic amino acid residue represented by formula (II-1) (in the formula, R³ is an optional substituent, and if present, it represents at least one substituent selected from the group consisting of a hydroxyl group, a carboxyl group, or a 1 to 5 carbon alkyl group; n represents an integer of 0 or 1, m represents an integer of 0 to 5, and ^{∗} represents a binding site; the above alkyl group may further have a substituent, and examples of the substituent include a hydroxyl group, an alkenyl group, an amino group, a carboxyl group, a sulfhydryl group, and the like) (in this case, the nitrogen atom bonded to R corresponds to the amino group of the aromatic amino acid). 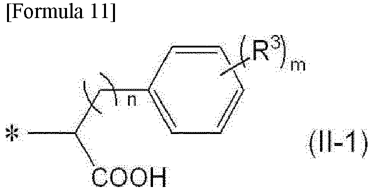 Examples of the aromatic amino acid residue represented by formula (II-1) include a group represented by the following structural formula. ^{∗} represents a binding site.

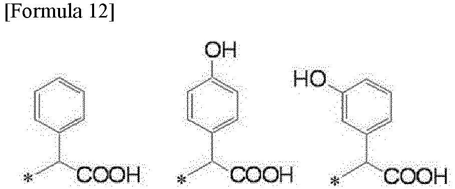

Examples of R in the formula include a heterocyclic group. The heterocyclic group is not particularly limited as long as it is a cyclic group containing a hetero atom, and it may be either an aliphatic heterocyclic group or an aromatic heterocyclic group. Moreover, examples of the hetero atom may include a nitrogen atom, an oxygen atom, a sulfur atom, and the like.

Among these, R in formula (I) is preferably a nitrogen-containing heterocyclic group containing a nitrogen atom as a hetero atom. More preferably, it is a heterocyclic group formed by removing one hydrogen atom from a heterocyclic compound selected from the group consisting of purine, pyridine, pyridazine, pyrrole, imidazole, pyrazole, and pyrazolone. Further preferably, it is a heterocyclic group formed by removing one hydrogen atom from a heterocyclic compound selected from the group consisting of purine, pyridine, and pyrazolone. Note that the position at which the hydrogen atom is removed from the heterocyclic compound is not particularly limited. Among these, it is preferable that the hydrogen atom bonded to the carbon atom of the heterocyclic compound is removed.

The heterocyclic group in R may have a substituent. Examples of the substituent in the heterocyclic group may include a 1 to 5 carbon alkyl group, an aryl group having a carbon number of no less than 6 and no more than 14, a carboxyl group, a carbamoyl group, a sulfonic acid group, and the like. Among these, the substituent in the heterocyclic group is preferably at least one selected from a phenyl group and a carbamoyl group.

The number of substituents in the heterocyclic group is not particularly limited, but it is preferably 0 to no more than 3.

Examples of R in formula (I) include a 2 to 8 carbon alkyl group. The 2 to 8 carbon alkyl group may be linear, branched, or cyclic. Among these, the 2 to 8 carbon alkyl group is preferably linear or branched, and it is more preferably linear. Also, the number of carbon atoms is preferably 2 to 6. Note that the number of carbon atoms of the alkyl group does not include the number of carbon atoms of substituents on the alkyl group.

The alkyl group in R may have a substituent. Examples of the substituent in the alkyl group may include a 1 to 5 carbon alkyl group, an aryl group having a carbon number of no less than 6 and no more than 14, an arylalkyl group having a carbon number of no less than 7 and no more than 16, a hydroxyl group, a carboxyl group, a carbamoyl group, a sulfonic acid group, an amino group, a carbamoyloxy group, a ureido group, a thioureido group, an alkyl sulfide group, an alkyl disulfide group, a group composed by removing R from the compound represented by formula (I), a group composed by removing one hydrogen atom from fluorescamine, and the like. Among these, it is preferably at least one selected from the group consisting of a hydroxyl group, a carboxyl group, an amino group, a carbamoyloxy group, a 7 to 14 carbon arylalkyl group, a thioureido group, a group composed by removing R from the compound represented by formula (I), and a group composed by removing one hydrogen atom from fluorescamine.

The number of substituents in the alkyl group is not particularly limited, but it is preferably 0 to no more than 3.

Further, the substituent in the alkyl group may further have a substituent if possible. The substituents contained in the substituents are the same as the substituents in the alkyl group.

Examples of R in formula (I) include an aryl group. The aryl group is preferably a 6 to 14 carbon aryl group, more preferably a 6 to 10 carbon aryl group, and further preferably a phenyl group.

The aryl group in R may have a substituent. Examples of the substituent in the aryl group may include a 1 to 5 carbon alkyl group, an aryl group having a carbon number of no less than 6 and no more than 14, a hydroxyl group, a carboxyl group, a sulfonic acid group, a carbamoyl group, an arylcarbonyl group, and the like. Among these, it is preferably at least one selected from the group consisting of a hydroxyl group, a carboxyl group, a sulfonic acid group, a carbamoyl group, and an arylcarbonyl group.

The number of substituents in the aryl group is not particularly limited, but it is preferably 0 to no more than 3.

Furthermore, the substituent in the aryl group may further have a substituent if possible. The substituents contained in the substituents are the same as the substituents in the aryl group. Furthermore, the substituents in the aryl group may be bonded to each other to form a cyclic structure if possible.

### [Method for Producing Compound Represented by Formula (I)]

The compound represented by formula (I) used in the present disclosure may be obtained by chemical synthesis or may be obtained by purifying from a culture of a filamentous fungus, for example, *Stachybotrys microspora* (*Stachybotrys microspora*). Examples of a method for purifying the compound represented by formula (I) from a culture of filamentous fungi include a method in which the compound of interest is purified from a culture obtained by adding a predetermined organic amino compound additive to a culture solution of *Stachybotrys microspora.* These methods are taught, for example, in JP 2004-224737 A, JP 2004-224738 A, WO 2007/111203, and the like.

The compound represented by formula (I) used in the present disclosure may be an enantiomer, a diastereomer, or a mixture of enantiomers or diastereomers. The enantiomer, diastereomer, and mixture of enantiomers or diastereomers may be obtained by chemical synthesis or by purification from a culture of filamentous fungi. When obtained by purification from a culture of filamentous fungi, isomers corresponding to each can be obtained using a D isomer or L isomer organic amino compound additive added to the filamentous fungi medium.

### <Compound Represented by Formula (IA)>

The compound represented by formula (I) is preferably a compound represented by the following formula (IA).

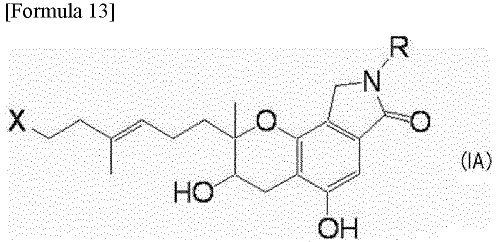

In formula (IA), X is -CHY-C(CH₃)₂Z, and Y and Z are each independently -H or -OH, or they form a single bond together. R represents a hydrogen atom or a substituent having a molecular weight of 1,000 or less.

R in formula (IA) is synonymous with R in formula (I), and preferable aspects are also the same.

### [Compound Represented by Formula (II)]

One specific example of the compound represented by formula (I) used in the present disclosure is the compound represented by the following formula (II).

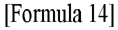

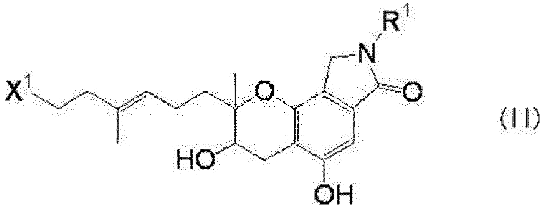

In formula (II), X¹ is -CHY-C(CH₃)₂Z, Y and Z are each independently -H or -OH, or they form a single bond together, and R¹ represents any one of the following (A) to (D):
(A) a residue obtained by removing a single amino group from an amino compound selected from the group consisting of natural amino acids, D isomers of natural amino acids, and compounds obtained by substituting at least one carboxyl group with a hydrogen atom, a hydroxyl group, or a hydroxymethyl group in natural amino acids and D isomers of natural amino acids (however, this excludes -(CH)₂-OH)
(B) an aromatic group having at least one selected from the group consisting of a carboxyl group, a hydroxyl group, a sulfonic acid group, and a secondary amino group as a substituent or as a part of a substituent, or an aromatic group containing a secondary amino group and optionally containing a nitrogen atom,
(C) an aromatic amino acid residue represented by formula (II-1) (in the formula, R³ are each independently and optionally a substituent, and if present, represent a hydroxy group, a carboxyl group, or a 1 to 5 carbon alkyl group, n represents an integer of 0 or 1, m represents an integer of 0 to 5, and ^{∗} represents a binding site)

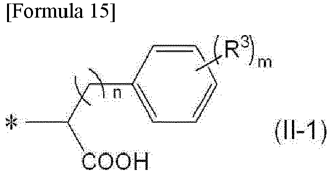

(D) a substituent represented by -L¹-L²-R⁴- (in the formula, L¹ represents a linking group which is a 1 to 4 carbon alkene group having a carboxyl group, L² represents a linking group represented by - NH-C(=O)- or -NH-C(=S)-NH-, and R⁴ is a 9-fluorenylalkoxy group having a 1 to 3 carbon alkoxy group or a polyheterocyclic group represented by formula (II-2) below).

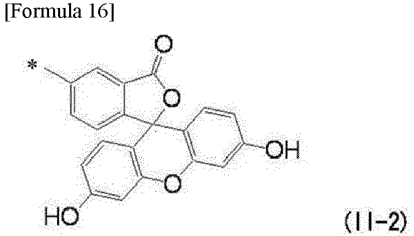

Compounds in which R¹ is the (A) in formula (II) will be described.

The (A) is a residue obtained by removing a single amino group from an amino compound selected from the group consisting of natural amino acids, D isomers of natural amino acids, and compounds obtained by substituting at least one carboxyl group with a hydrogen atom, a hydroxy group, or a hydroxymethyl group in natural amino acids and D isomers of natural amino acids (however, this excludes -(CH)₂-OH).

The natural amino acid is not particularly limited as long as it is a naturally occurring amino acid, and examples thereof include an α-amino acid, a β-amino acid, a γ-amino acid, a δ-amino acid, and the like. Such amino acids may be obtained from natural products or may be artificially obtained by a method such as organic synthesis.

Examples of the natural amino acid include glycine, alanine, threonine, valine, isoleucine, tyrosine, cysteine, cystine, methionine, histidine, aspartic acid, glutamic acid, asparagine, glutamine, arginine, lysine, hydroxylysine, ornithine, citrulline, homocysteine, 3,4-dihydroxyphenylalanine, homocystine, diaminopimelic acid, diaminopropionic acid, serine, leucine, phenylalanine, tryptophan, and the like as the α-amino acids. Examples of the β-amino acid include β-alanine and the like. Examples of the γ-amino acid include γ-aminobutyric acid, carnitine, and the like. Examples of the δ-amino acid include 5-aminolevulinic acid, 5-aminovaleric acid, and the like.

Examples of the natural amino acid or D isomer of a natural amino acid wherein at least one carboxyl group is substituted with a hydrogen atom, a hydroxyl group, or a hydroxymethyl group include amino alcohols and amines. Examples of such amino alcohols include 2-aminoethanol.

Specific examples of compounds in which R¹ is the (A) in formula (II) are the compounds shown in Table 1 below. Note, the "added organic amino compound" in the table indicates an added organic amino compound to be used when the compound is purified from a culture product obtained by adding a predetermined added organic amino compound to a culture solution of *Stachybotrys microspora* (the same is true hereinafter). In the table, ^{∗} represents a binding site (the same is true hereinafter).

**[Table 1]**

| 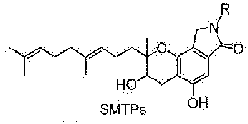 | | | |
|---|---|---|---|
| Compound No. | Mol. Wt. | R= | Added Organic Amino Compound |
| SMTP-3 | 4736 | 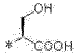 | L-Serine |
| SMTP-4 | 5337 | 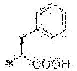 | L-Phenylalanine |
| SMTP-4Me | 547 7 | 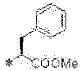 | L-Phenylalanine methyl ester |
| SMTP-4D | 533.7 | 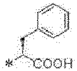 | D-Phenylalanine |
| SMTP-5 | 499.6 | 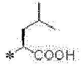 | L-Leucine |
| SMTP-5D | 499.6 | 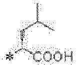 | D-Leucine |
| SMTP-6 | 572.7 | 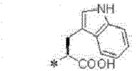 | L- Tryptophan |
| SMTP-6D | 572.7 | 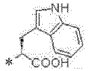 | D-Tryptophan |
| SMTP-10 | 499,6 | 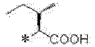 | L-Isoleucine |
| SMTP-11 | 485.6 | 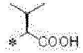 | L-Valine |
| SMTP-12 | 457.6 | 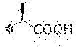 | L-Glycine |
| SMTP-13 | 517.7 | 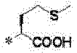 | L-Methionine |
| SMTP-14 | 549.7 | 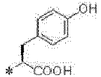 | L-Tyrosine |
| SMTP-15 | 542.7 | 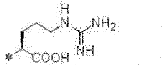 | L-Arginine |

The compounds shown in Table 1 above may be suitably used as the compound represented by formula (I) used in the present disclosure.

Compounds in which R¹ is the (B) in formula (II) will be described.

The (B) is an aromatic group having at least one selected from the group consisting of a carboxyl group, a hydroxyl group, a sulfonic acid group, and a secondary amino group as a substituent or as a part of a substituent, or an aromatic group containing a secondary amino group and optionally containing a nitrogen atom

Examples of the aromatic group include groups represented by the following structural formulas. In each structural formula, ^{∗} represents a binding site.

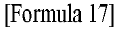

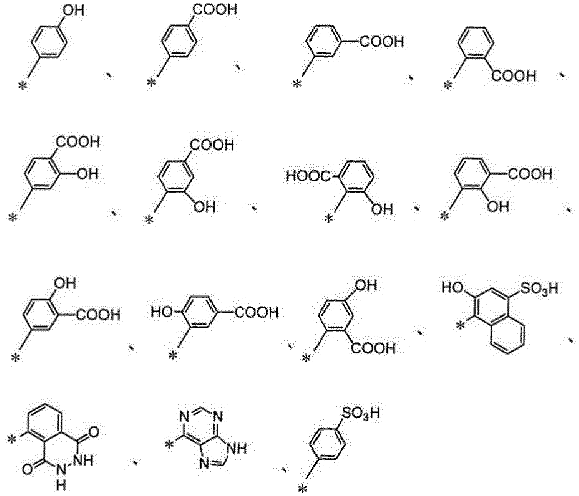

Specific examples of compounds in which R¹ is the (B) in formula (II) are the compounds shown in Table 2 below.

**[Table 2]**

| 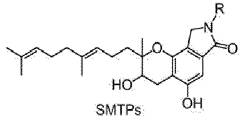 | | | |
|---|---|---|---|
| Compound No. | Mol. Wt. | I R= | Added Organic Amino Compound |
| SMTP-18 | 477.6 | 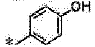 | p-Aminophenol |
| SMTP-19 | 505.6 | 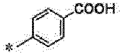 | p-Aminobenzoic acid |
| SMTP-20 | 505.6 | 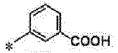 | m-Aminobenzoic acid |
| SMTP-21 | 505.6 | 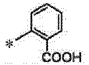 | o-Aminobenzoic acid |
| SMTP-22 | 5216 | 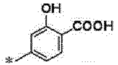 | 4-Aminosalicylic acid |
| SMTP-23 | 5216 | 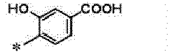 | 4-Amino-3-hydroxybenzoic acid |
| SMTP-24 | 521.6 | 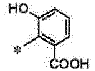 | 3 -Hydroxyanthranillic acid |
| SMTP-25 | 5216 | 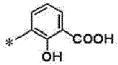 | 3 -Aminosalicylic acid |
| SMTP-26 | 521.6 | 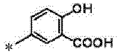 | 5-Aminosalicylic acid |
| SMTP-27 | 521.6 | 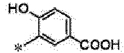 | 3-Amino-4-hydroxybenzoic acid |
| SMTP-28 | 521.6 | 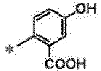 | 5-Hydroxyanthranillic acid |
| SMTP-32 | 503.6 | 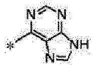 | Adenine or adenosine |
| SMTP-36 | 545.3 | 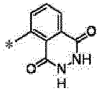 | 5-Amino-2,3-dihydro-1,4-phthalazinedione |
| SMTP-37 | 607.7 | 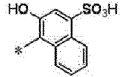 | 1 -Amino-2-naphthol-4-sulfonic acid |
| SMTP-42 | 541.7 | 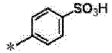 | p-Sulfanilic acid |

The compounds shown in Table 2 above may be suitably used as the compound represented by formula (I) used in the present disclosure.

A compound in which R¹ is the (C) in formula (II) will be described.

The (C) is an aromatic amino acid residue represented by formula (II-1) below (in the formula, R³ is an optional substituent, and if present, represents at least one substituent selected from the group consisting of a hydroxyl group, a carboxyl group, and a 1 to 5 carbon alkyl group; n represents an integer of 0 or 1, m represents an integer from 0 to 5, and ^{∗} represents a binding site; the foregoing alkyl group may have a further substituent, and examples of substituents include a hydroxyl group, an alkenyl group, an amino group, a carboxyl group, a sulfhydryl group, and the like) (in this case, a nitrogen atom bound to R¹ corresponds to an amino group of an aromatic amino acid).

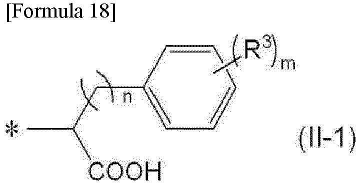

Examples of the aromatic amino acid residue represented by formula (II-1) include a group represented by the following structural formula. ^{∗} represents a binding site.

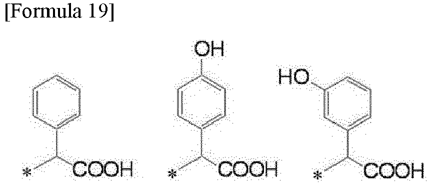

Specific examples of compounds in which R¹ is the (C) in formula (II) are the compounds shown in Table 3 below.

**[Table 3]**

| 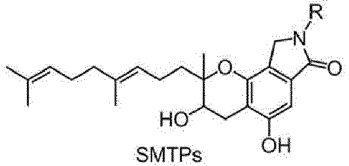 | | | |
|---|---|---|---|
| Compound No. | Mol. Wt. | R = | Added Organic Amino Compound |
| SMTP-43 | 519.6 | 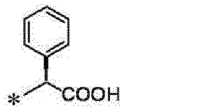 | L-Phenylglycine |
| SMTP-43D | 519.6 | 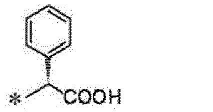 | D-Phenylglycine |
| SMTP-44 | 535.6 | 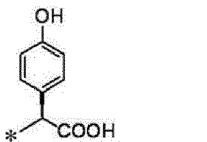 | L-4-Hydroxy phenylglycine |
| SMTP-44D | 535.6 | 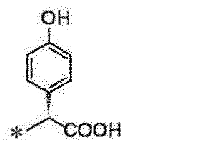 | D-4-Hydroxy phenylglycine |
| SMTP-45-I | 535.6 | 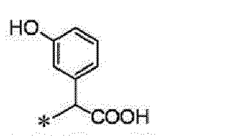 | DL-3-Hydroxy phenylglycine |
| SMTP-45-II | 535.6 | 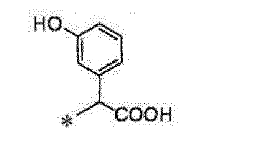 | DL-3-Hydroxy phenylglycine |

The compounds shown in Table 3 above may be suitably used as the compound represented by formula (I) used in the present disclosure.

A compound in which R¹ is the (D) in formula (II) will be described.

The (D) is a substituent represented by -L¹-L²-R⁴- (in the formula, L¹ represents a linking group which is a 1 to 4 carbon alkene group having a carboxy group, L² represents a linking group represented by -NH-C(=O)- or -NH-C(=S)-NH-, and R⁴ is a 9-fluorenylalkoxy group having a 1 to 3 carbon alkoxy group or a polyheterocyclic group represented by formula (II-2) below).

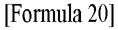

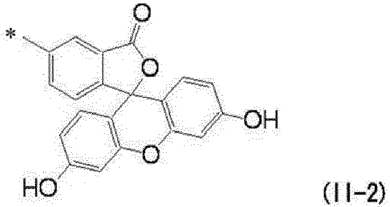

Specific examples of compounds in which R¹ is the (D) in formula (II) are the compounds shown in Table 4 below.

**[Table 4]**

| 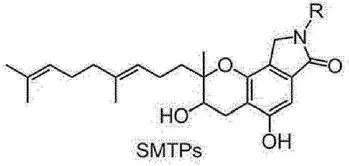 | | | |
|---|---|---|---|
| Compound No. | Mol. Wt. | R = | Added Organic Amino Compound |
| SMTP-46 | 722.9 | 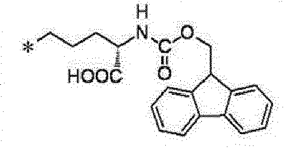 | Nα-Fmoc-L-ornithine |
| SMTP-47 | 722.9 | 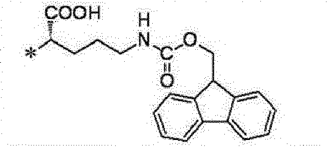 | Nδ-Fmoc-L-ornithine |
| SMTP-48 | 890.0 | 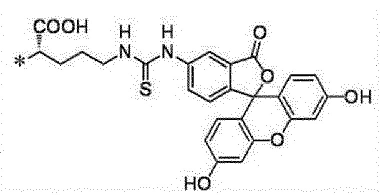 | Nδ-FITC-L-ornithine |
| SMTP-49 | 890.0 | 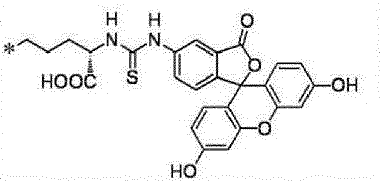 | Nα-FITC-L-ornithine |

The compounds shown in Table 4 above may be suitably used as the compound represented by formula (I) used in the present disclosure.

### [Compound Represented by Formula (III)]

One specific example of the compound represented by formula (I) used in the present disclosure is the compound represented by formula (III) below.

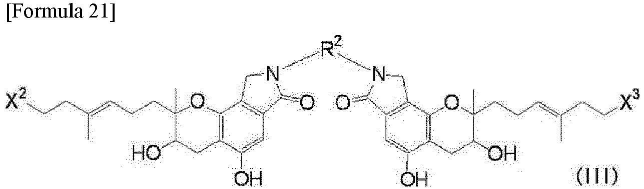

In the formula (III), X² and X³ are each independently -CHY-C(CH₃)₂Z, and Y and Z are each independently -H or -OH, or they form a single bond together. R² represents a residue obtained by removing two amino groups from an amino compound selected from the groups consisting of: natural amino acids having two amino groups, D isomers of natural amino acids having two amino groups, compounds in which at least one carboxyl group in a natural amino acid having two amino groups or a D isomer of a natural amino acid having two amino groups is replaced with a hydrogen atom, a hydroxyl or a hydroxymethyl group, and compounds represented by H₂N-CH(COOH)-(CH₂)ₙ-NH₂ (n is an integer from 0 to 9) and H₂N-CH(COOH)-(CH₂)ₘ-Sₚ-(CH₂)_{q}-CH(COOH)-NH₂ (m, p and q are each independently integers from 0 to 9).

The n represents an integer from 0 to 9, preferably an integer from 0 to 6, more preferably an integer from 1 to 5, and further preferably an integer from 1 to 4.

The m represents an integer from 0 to 9, preferably an integer from 0 to 4, more preferably an integer from 1 to 3, and further preferably an integer of 1 or 2.

The p represents an integer from 0 to 9, preferably an integer from 0 to 4, more preferably an integer from 1 to 3, and further preferably an integer of 1 or 2.

The q represents an integer from 0 to 9, preferably an integer from 0 to 4, more preferably an integer from 1 to 3, and further preferably an integer of 1 or 2.

When the p is 0, m+q is preferably an integer from 0 to 9, more preferably an integer from 0 to 6, further preferably an integer from 1 to 5, and particularly preferably an integer from 1 to 4.

Examples of natural amino acids having two amino groups include hydroxylysine, citrulline, cystine, homocystine, diaminopimelic acid, diaminopropionic acid, lysine, ornithine, and the like as α-amino acids.

Examples of compounds in which at least one carboxyl group in a natural amino acid having two amino groups or a D isomer of a natural amino acid having two amino groups is replaced with a hydrogen atom, a hydroxyl group, or a hydroxymethyl group include H₂N-(CH₂)ₖ-NH₂ (k is an integer from 1 to 10, preferably an integer from 1 to 6, and more preferably an integer from 1 to 4).

Specific examples of compounds represented by formula (III) include the compounds shown in Table 5 below.

**[Table 5]**

| 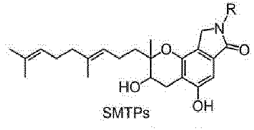 | | | |
|---|---|---|---|
| Compound No. | Mol. Wt. | R = | Added Organic Amino Compound |
| SMTP-7 | 869.1 | 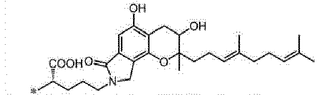 | L-Ornithine |
| SMTP-7D | 869.1 | 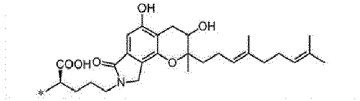 | D-Ornithine |
| SMTP-8 | 883.1 | 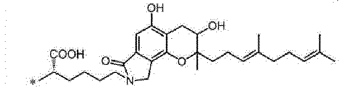 | L-Lysine |
| SMTP-8D | 883.1 | 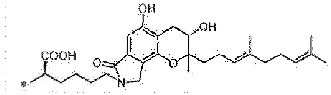 | D-Lysine |
| SMTP-9 | 977.2 | 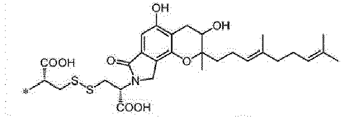 | D-Cysteine |
| SMTP-29 | 839.1 | 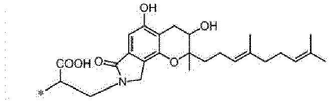 | DL-2,3-Diaminopropionic acid |
| SMTP-31 | 925.2 | 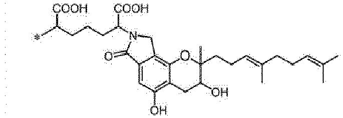 | DL-2,6-Diaminopropionic acid |

The compounds shown in Table 5 above may be suitably used as the compound represented by formula (I) used in the present disclosure.

For the compound represented by formula (I) used in the present disclosure, the compound represented by (I) is preferably a compound represented by formula (II) or formula (III) below. In addition to the compound represented by the formula (II) or (III), specific examples of the compound represented by formula (I) are compounds shown in Table 6 to Table 8 below.

**[Table 6]**

| 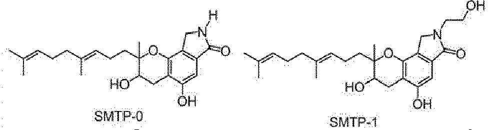 | | |
|---|---|---|
| Compound No. | Mol. Wt. | Added Organic Amino Compound |
| SMTP-0 | 385.5 | Ammonium chloride |
| SMTP-1 | 429 6 | 2-Aminoethanol |

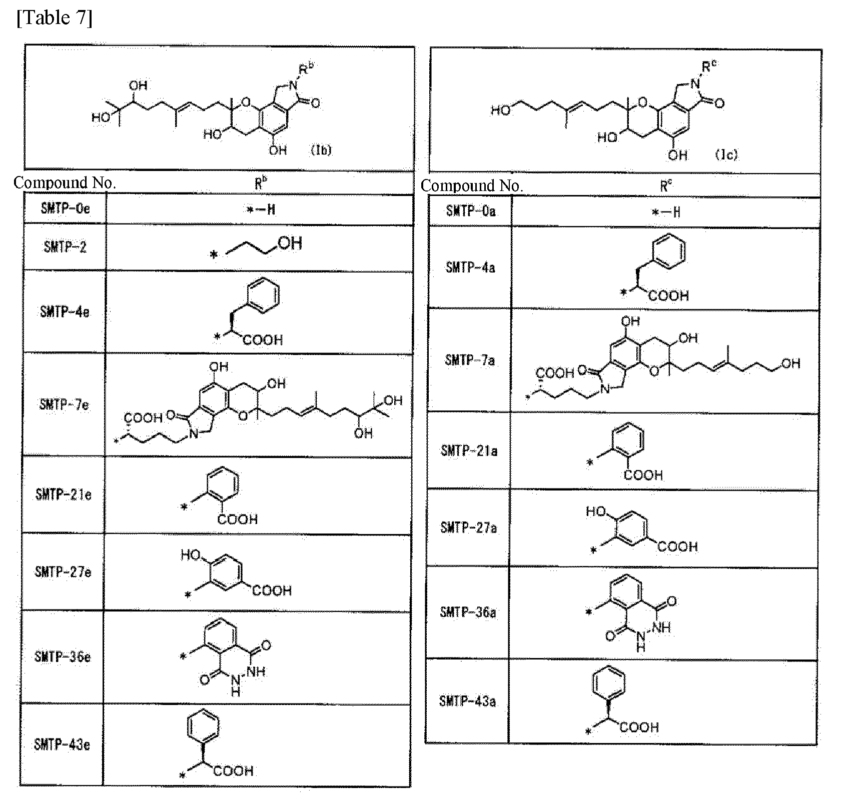

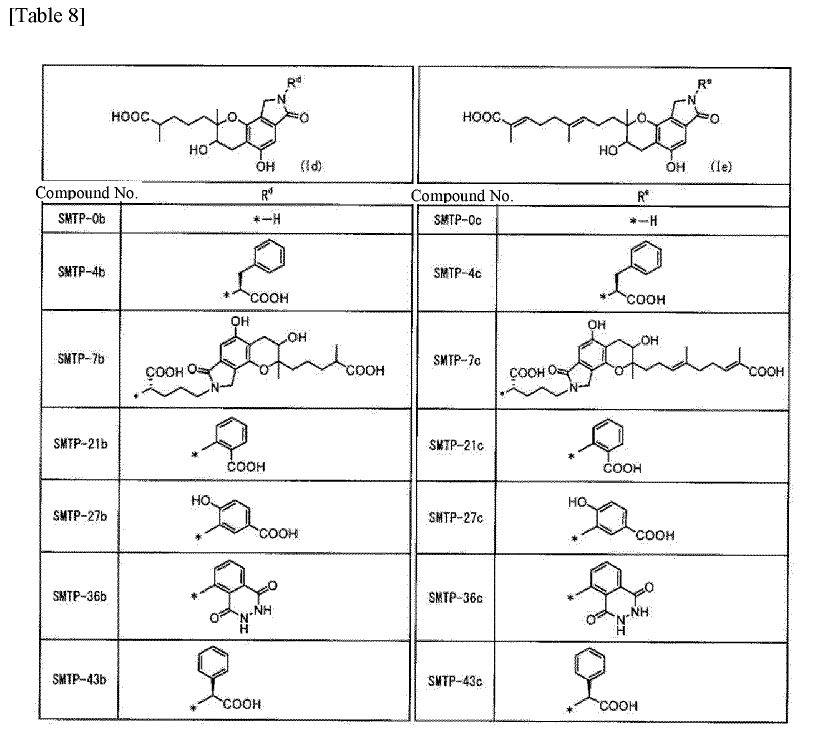

The compounds shown in Table 6 to Table 8 above may be suitably used as the compound represented by formula (I) included in a drug.

Among the compounds described above, the compound represented by formula (I) preferably includes at least one selected from the group consisting of SMTP-0, SMTP-1, SMTP-4, SMTP-5D, SMTP-6, SMTP-7, SMTP-8, SMTP-11 to 14, SMTP-18 to 29, SMTP-36, SMTP-37, SMTP-42, SMTP-43, SMTP-43D, SMTP-44, SMTP-44D, SMTP-46, and SMTP-47. Among the compounds described above, the compound represented by formula (I) further preferably includes SMTP-27 or SMTP-44D The compound represented by formula (I) may contain either SMTP-27 or SMTP-44D or may contain both SMTP-27 and SMTP-44D.

The compound represented by formula (I) used in the present disclosure is contained in the drug in a free form, a pharmaceutically acceptable salt or ester form, or a solvate form. An inorganic acid or organic acid is suitable for forming a pharmaceutically acceptable salt of a compound represented by formula (I) used in the present disclosure. Examples of the inorganic acid include hydrochloric acid, hydrobromic acid, sulfuric acid, nitric acid, phosphoric acid, and the like. Examples of the organic acid include citric acid, formic acid, fumaric acid, malic acid, acetic acid, succinic acid, tartaric acid, methanesulfonic acid, p-toluenesulfonic acid, and the like.

Furthermore, for example, an alkali metal such as sodium, potassium, calcium, or magnesium; a compound containing an alkaline earth metal; a basic amine; or a basic amino acid is also suitable for forming a pharmaceutically acceptable salt of a compound represented by formula (I) used in the present disclosure.

Moreover, a 1 to 10 carbon alcohol, carboxylic acid, or the like, and preferably methyl alcohol, ethyl alcohol, acetic acid, propionic acid, or the like is suitable for the formation of a pharmaceutically acceptable ester of the compound represented by formula (I) used in the present disclosure.

Furthermore, water and the like are suitable for forming a pharmaceutically acceptable solvate of a compound represented by formula (I) used in the present disclosure.

The foregoing description of specific examples of the compound represented by formula (I) such as SMTP-27 or SMTP-44D also includes these forms such as salts.

### <Carriers and Additives>

The type of carriers and formulation additives used for preparation of the drug of the present disclosure is not particularly limited. The drug of the present disclosure may be formulated using a compound represented by formula (I) of the present disclosure, a pharmaceutically acceptable solid carrier (for example, gelatin or lactose), or a liquid carrier (for example, water, saline or glucose aqueous solution).

The drug of the present disclosure may be used as a pharmaceutical composition. The type of formulation additives used in pharmaceutical compositions including the drug of the present disclosure is not particularly limited. The pharmaceutical composition including the drug of the present disclosure may be a pharmaceutical composition including a compound represented by formula (I) of the present disclosure, a pharmaceutically acceptable solid carrier (for example, gelatin or lactose), or a liquid carrier (for example, water, saline or glucose aqueous solution).

### <Dosage>

The dosage also depends on the type of compounds used as active ingredients, severity of diabetes complications, and the like, but an effective amount of the drug of the present disclosure per kg of body weight per dose in an adult is preferably 0.001 mg/kg to 200 mg/kg, more preferably 0.01 mg/kg to 60 mg/kg, and further preferably 0.1 mg/kg to 50 mg/kg. The number of times of administration is not particularly limited, and the drug may be used for one administration, may be used for repeated administration, or may be used for continuous administration. The interval of administration and period of administration may be selected by a person skilled in the art according to clinical findings, imaging, blood findings, comorbid diseases, previous history, or the like. Note, in the present disclosure, for example, when the effective amount or dosage per kg of body weight is 0.001 mg, the effective amount or dosage may be expressed as "0.001 mg/kg" or "0.001 mg/kg of body weight."

When using the drug of the present disclosure in repeated administration, sustained administration may be performed up to 24 hours per day from the viewpoint that the affected part continuously contacts the drug of the present disclosure.

The method of administration is not particularly limited, and various administration routes can be selected, such as intravenous administration, subcutaneous administration, intramuscular administration, or oral administration. For example, in the acute phase of each disease, intravenous administration, specifically intravenous injection or intravenous drip, can be used from the viewpoint of rapidly and surely administering a desired dose to a patient. In this case, for example, 10% of 1 dose may be rapidly injected, and 90% of 1 dose may be injected over 30 minutes to 1 hour.

### <Use>

This drug of the present disclosure is a drug used for diabetes complications. The drug of the present disclosure may be used for two or more diabetes complications described below.

Examples of diabetes complications include diabetic neuropathy, diabetic kidney disease, diabetic nephropathy, diabetic retinopathy, arteriosclerosis, diabetic foot lesions, cerebral infarctions, myocardial infarctions, and the like.

Among diabetes complications, the drug of the present disclosure may be suitably used for diabetic neuropathy and diabetic kidney disease.

Furthermore, the above diabetic neuropathy includes polyneuropathy and mononeuropathy. Examples of polyneuropathy include sensory neuropathy, motor neuropathy, and autonomic disorder, but the drug of the present disclosure may also be suitably used for any diabetic neuropathy.

Additionally, diabetic nephropathy is one example of the diabetic kidney disease. Diabetic nephropathy in the present disclosure may mean a disease satisfying any one condition selected from the group consisting of: eGFR value (mL/min/1.73 m²), which is the converted glomerular filtration rate calculated from serum creatinine level, age, and gender, being 30 or more and less than 90 and urinary albumin value (mg/gCr) being less than 30, eGFR value (mL/min/1.73 m²) being 30 or more and less than 90 and urinary albumin value (mg/gCr) being 30 or more, and eGFR value (mL/min/1.73 m²) being less than 30. The drug of the present disclosure may be suitably used for any diabetic kidney disease. Note, urine albumin level may be calculated by urine albumin (g/dL) / creatinine (mg/dL), and urine albumin volume may be measured using an immunoturbidimetric method, a latex coagulation method, or the like. Male eGFR level may be calculated from 194 × (serum creatinine value^{-1.094}) × (age^{-0.287}). Female eGFR level may be calculated from 194 × (serum creatinine value^{-1.094}) × (age^{-0.287}) × 0.739. Serum creatinine level may be measured using a commercially available kit such as LabAssay (trademark) Creatinine, an enzyme method, or the Jaffe method.

The drug of the present disclosure may be used for the treatment or prevention of diabetes complications, but it is more preferably used for the treatment or prevention of diabetic neuropathy or diabetic kidney disease.

In the present disclosure, "treatment" may be used to improve or suppress symptoms, and suppression of increased severity and reduction or mitigation of symptoms are also included in this term.

In the present disclosure, "prevention" means inhibition of onset, reduction of risk of onset, delay of onset, and the like.

In the present disclosure, use for diabetes complications refers to use when symptoms due to diabetes complications are found and when it is predicted that symptoms due to diabetes complications will be manifested.

The drug of the present disclosure is used to treat symptoms due to diabetes complications, to suppress the progress of the symptoms, or to mitigate the symptoms. However, the drug of the present disclosure is used in combination depending on the time of use or the symptoms at the time of use and is not to be interpreted in a limiting manner.

Examples of when symptoms due to diabetes complications are found or when it is predicted that symptoms due to diabetes complications will be manifested may include during treatment of diabetes or after treatment. Additionally, this also includes when high blood glucose is observed even when urinary sugar is not observed (so-called urinary sugar negative).

Note, when there is a possibility of diabetes complications, use is possible without being limited to the times described above.

In the present Specification, "high blood glucose" refers to satisfying any of group A, satisfying group B, or satisfying any of group A and satisfying group B. Alternatively, it refers to satisfying group C and any of group D. Alternatively, it refers to satisfying both of group E.
Group A
   - Fasting blood glucose level of 126 mg/dL or more
   - In 75 g oral glucose tolerance test (OGTT), blood glucose level is 200 mg/dL or more 2 hours after sugar load
   - Casual blood glucose level of 200 mg/dL or more
Group B
   - Hemoglobin A1c (HaA1c) is 6.5% or more of total hemoglobin
Group C
   - Hemoglobin A1c (HbA1c) is less than 6.5% of total hemoglobin
Group D
   - Fasting blood glucose level of 110 mg/dL to 125 mg/dL
   - In 75 g OGTT, blood glucose level is 140 mg/dL to 199 mg/dL 2 hours after sugar load Group E
   - Fasting blood glucose of 100 mg/dL to 109 mg/dL
   - HbA1c is 5.6% to 5.9% of total hemoglobin

Furthermore, the diabetic neuropathy or diabetic kidney disease includes diabetic neuropathy or diabetic kidney disease caused by type 1 diabetes and diabetic neuropathy or diabetic kidney disease caused by type 2 diabetes. The drug of the present disclosure may be used for any of these, but it may be suitably used for diabetic neuropathy or diabetic kidney disease due to type 2 diabetes.

The drug of the present disclosure may be used prophylactically even when a diabetes complication other than diabetic neuropathy or diabetic kidney disease occurs.

The drug of the present disclosure may be used without being limited to human use. As other applicable subjects, the drug may be used for non-human animals such as livestock such as cattle, horses, and sheep and pets such as dogs, cats, and monkeys.

### <Combination With Other Drugs>

The drug of the present disclosure may be used alone or together with another drug used for at least one or more diabetes complications.

Examples of other drugs used for diabetes complications include drugs for controlling blood glucose levels, drugs for suppressing acceleration of polyol metabolic activity, non-steroidal anti-inflammatory drugs (NSAIDs), tricyclic antidepressants, anticonvulsants, and drugs used for conventionally known diabetes complications such as antiarrhythmic drugs or drugs for treating neuropathic pain.

Therapeutic effect can be expected to be enhanced by using the drug of the present disclosure and other drugs in combination. In this case, the drug of the present disclosure may be used simultaneously with other drugs or at a different time.

The drug of the present disclosure may be configured as a pharmaceutical composition included in the drug in a free form, a pharmaceutically acceptable salt or ester form, or a solvate form.

### (Method of Treatment or Prevention)

The method for treatment or prevention of the present disclosure is preferably a method for treatment or prevention that uses a drug used for treating or preventing a diabetes complication.

The method for treatment or prevention of the present disclosure is a method for treating or preventing a diabetes complication in a subject having a diabetes complication or at risk of developing a diabetes complication, including administering an amount of the drug of the present disclosure effective for treatment or prevention.

For example, effects such as suppression of increased severity of diabetes complications, reduction or mitigation of symptoms, inhibition of the onset of diabetes complications, reduced risk of onset, or delayed onset can be obtained by the treatment method or prevention method of the present disclosure.

The aspects of the dosage, interval of administration, period of administration, and method of administration of the drug of the present disclosure in the treatment method or prevention method of the present disclosure are the same as those already described for the drug of the present disclosure.

The treatment method or prevention method of the present disclosure is applicable to any of the diabetes complications above.

### (Compound)

Another aspect of the present disclosure is preferably a drug used for the treatment or prevention of diabetes complications, and the compound described above represented by the foregoing formula (I) is used for the treatment or prevention of diabetes complications. Additionally, another mode of the present disclosure is use of a compound of the foregoing formula (I) in manufacturing a therapeutic drug or prophylactic drug for diabetes complications.

The details of use and the like for treating or preventing diabetes complications are the same as described above for the method for treating or preventing diabetes complications, and favorable aspects are the same.

### Examples

Examples of the present disclosure will be described below, but the present disclosure is not limited thereto. Unless otherwise stated, the mass basis is "%."

### <Preparation of SMTP-27, SMTP-44D, Edaravone, Pregabalin, and Metformin>

Production of SMTP-27 or SMTP-44D was carried out using the method taught in JP 2004-224738 A. A culture product obtained when adding 3-amino-4-hydroxybenzoic acid or D-4-hydroxyphenylglycine as an added organic amino compound to a culture medium of a *Stachybotrys microspora* IFO30018 strain was purified to obtain SMTP-27 or SMTP-44D. A 50 mg/mL solution was prepared by adding 0.3 N (0.3 mol/L) NaOH aqueous solution and saline solution (0.9% NaCl in water) to the dried solid of SMTP-27 or SMTP-44D obtained by purification. Then, a 0.3 N (0.3 mol/L) HCl aqueous solution and physiological saline were used to adjust the concentration of SMTP-27 or SMTP-44D to 10 mg/mL and the pH to be weakly alkaline, filtration sterilization was performed, and this was divided into small groups and freeze-preserved at - 30°C. SMTP-27 or SMTP-44D was diluted with saline for use as needed.

The freeze-preserved SMTP-27 or SMTP-44D was dissolved in physiological saline to 1 mg/mL immediately prior to testing.

A 1.5 mg/mL stock solution of edaravone (product name: Radicut, Mitsubishi Tanabe Pharma Corporation) was used. 25 mg tablets of pregabalin (product name: Lyrica OD tablets 25 mg, Pfizer Japan Inc.) were used. A raw material powder of metformin (Wako Pure Chemical Industries, Ltd.) was used. The drugs above were prepared and diluted using physiological saline as needed.

### (Example 1: Test Using Diabetic Neuropathy Model Animal)

### <Preparation of Diabetic Neuropathy Model Animal>

9-week-old C57BL/6J male mice were purchased and familiarized with an animal facility for 1 week. 200 mg/kg of streptozotocin (streptozotocin; STZ) was administered subcutaneously into the peritoneal cavity of 10-week-old mice to prepare streptozotocin-induced diabetic mice (hereinafter also referred to as "STZ administration week 0"). One week after the administration of streptozotocin, blood was collected from the tail vein of the mice, and the blood glucose level (BS) was measured using an automatic blood glucose measuring device (product name: Medisafe (trademark) Mini GR-102, made by Terumo (Co)). Mice having a blood glucose level of 400 mg/dL or more were determined to be diabetic mice.

Note, the administration date of streptozotocin (STZ) was set as week 0.

### «Preparation of Non-Diabetic Mice»

200 mg/kg of physiological saline was administered subcutaneously into the peritoneal cavity of 10-week-old C57BL/6J male mice familiarized for 1 week. Non-diabetic mouse were otherwise prepared in the same manner as the diabetic mice.

### «Administration of Drug»

Twelve per group of the diabetic mice prepared above were randomly assigned to each of an STZ group, an SMTP-44D (0.3 mg/kg) group, an SMTP-44D (3 mg/kg) group, an SMTP-44D (30 mg/kg) group, an edaravone (edaravone) group, and a pregabalin (pregabalin) group. Furthermore, 12 non-diabetic mice were used as a control group. Note, "SMTP-44D groups" in the following sections collectively refers to the three groups of SMTP-44D (0.3 mg/kg), SMTP-44D (3 mg/kg), and SMTP-44D (30 mg/kg).

The SMTP-44D groups, the edaravone group, and the pregabalin group were administered SMTP-44D (0.3 mg/kg, 3 mg/kg, or 30 mg/kg doses, respectively), edaravone (10 mg/kg dose), or pregabalin (10mg/kg dose) into the peritoneal cavity once a day for 3 consecutive weeks from the day following the first week to the fourth week after STZ administration.

The STZ group was administered saline into the peritoneal cavity once a day for 3 consecutive weeks from the day following the first week to the fourth week after STZ administration. The control group was administered physiological saline into the peritoneal cavity once a day for 3 consecutive weeks at the same time as the administration of physiological saline for the STZ group.

For the above-mentioned STZ group, SMTP-44D groups, edaravone group, pregabalin group, and control group mice, the following measurements were conducted every week from the 0th week, which began on the day when streptozotocin (STZ) was administered, to the 4th week (that is, a total of five times). As algesia tests, the following von Frey test and hot plate test were conducted, and hind plantar blood flow, body weight (BW), and blood glucose level (BS) were measured using the above-mentioned automatic blood glucose measure device. Note, for example, the 0th week represents the 0th day. That is, the measurements were conducted on the 0th, 7th, 14th, 21st, and 28th days.

The measurement results of the body weights (BW) and blood glucose levels (BS) are described in Table 9. Moreover, sciatic nerve blood flow, sciatic nerve conduction velocity, thickness and G-ratio of myelin in the sciatic nerves, oxidative stress (malondialdehyde: MDA), and inflammatory cytokines (TNF-α, IL-1β, and IL-6) for the above-mentioned STZ group, SMTP-44D groups, edaravone group, pregabalin group, and control group mice in the 4th week.

### <Evaluations>

### [Von Frey test: evaluating mechanical stimulus]

The above-mentioned STZ group, SMTP-44D groups, edaravone group, pregabalin group, and control group mice were put in grid-like boxes, respectively, and were familiarized for one hour. After the familiarization, by using a Dynamic Plantar Aesthesiometer (tactometer) (manufactured by UGO BASILE Inc.), 1 g/s (that, pressurization increased by 1 g per second) of stimulation was imparted to the hind legs of these mice to measure mechanical thresholds (g) from the start of stimulation to the time when the mice withdrew their legs. The measurements were conducted for both hind legs three times at intervals of three minutes or longer, respectively. A mean value of three mechanical thresholds (g) was calculated for 1 mouse, and then a mean value of the 12 mice for the respective groups was found.

The measurement results are described in Table 10.

### [Hot plate test: evaluating thermal stimulus]

The above-mentioned STZ group, SMTP-44D groups, edaravone group, pregabalin group, and control group mice were put in grid-like boxes, respectively, and were left for 30 minutes. After having been left, an acrylic disc (diameter: 20 cm; height (that is, thickness of the acrylic disc): 25 cm) was placed on an EC hot plate (model number: EC1200) maintained at 49 to 50°C, and the mice were put on the acrylic disc. The measurements were started after the mice landed on the EC hot plate. The times from the mice landing to their action to jump or lick their hind legs were evaluated as thermal thresholds (seconds: s).

Note, to avoid burns on their plantae, the measurements were ceased 30 seconds after beginning. The measurements were conducted three times at 30-minute intervals. A mean value of three thermal thresholds (seconds: s) was calculated for 1 mouse, and then a mean value of the 12 mice for the respective groups was found.

The measurement results are described in Table 10.

### [Measuring hind plantar blood flow]

The hind plantar blood flows (perfusion unit: PU) of the above-mentioned STZ group, SMTP-44D groups, edaravone group, pregabalin group, and control group mice were measured under isoflurane anesthesia using a full-field laser perfusion imager.

The measurement results are described in Table 10.

### [Measuring sciatic nerve blood flow and sciatic nerve conduction velocity]

Sciatic nerves of the above-mentioned STZ group, SMTP-44D groups, edaravone group, pregabalin group, and control group mice on the 4th week were exposed under isoflurane anesthesia, and then, using the full-field laser perfusion imager, their sciatic nerve blood flows (perfusion unit: PU) were measured.

After the sciatic nerve blood flows were measured, a voltage was output from a PowerLab through the ER-1 Extracellular Amplifier to impart an electrical stimulus to the sciatic nerves using needle electrodes. The electrical stimulus went through the Analog Stimulus Isolator via recording electrodes attached to the gastrocnemius and was input into the PowerLab, and thus neuromuscular function response curves were measured.

The sciatic nerves were stimulated between two points at 8-mm intervals. The sciatic nerve conduction velocity (m/s) was calculated by dividing the latent difference of the respectively derived neuromuscular function response curves by the distance between the two points.

The sciatic nerve blood flow and sciatic nerve conduction velocity were measured using a Hot-1 small-sized heating controller and small-sized body temperature retention device (model number: HEATINGPAD-1), maintaining the rectal temperature at 37°C.

The measurement results are described in Table 11.

### [Measuring thickness and G-ratio of myelin in the sciatic nerve]

The above-mentioned STZ group, SMTP-44D (30 mg/kg) group, edaravone group, pregabalin group, and control group mice in the 4th week were dissected to remove the sciatic nerves. The sciatic nerves were fixed using neutral buffered formalin and embedded using an Epon resin, and then semi-thin segments were formed and dyed using toluidine blue. The cross sections of the sciatic nerves in the respective specimens were examined using a microscope equipped with a digital camera at 400x magnification. The thickness of myelin ((diameter of axon including myelin - diameter of axon)/2) and G-ratio (diameter of axon / diameter of axon including myelin) were measured.

The results are described in Table 11.

### [Measuring oxidative stress]

The sciatic nerves of the above-mentioned STZ group, SMTP-44D group (30 mg/kg), edaravone group, pregabalin group, and control group mice in the 4th week were homogenized using an RIPA buffer containing a protease inhibitor cocktail. Moreover, centrifugation was conducted at 4°C for 15 minutes at 1600 g, and the supernatant was collected and used for measuring MDA (µM) and total protein concentration (µg/mL), respectively. MDA was measured using a TBARS Assay Kit. The total protein concentration was measured using a Pierce (Trademark) BCA Protein Assay Kit. Oxidative stress levels (nmol/mg protein) were calculated from MDA (µM) and the total protein concentration (µg/mL).

The results are described in Table 11.

### [Measuring inflammatory cytokines]

The sciatic nerves of the above-mentioned STZ group, SMTP-44D group (30 mg/kg), edaravone group, pregabalin group, and control group mice on the 4th week were homogenized using the RIPA buffer containing a protease inhibitor cocktail. Moreover, centrifugation was conducted at 4°C for 15 minutes at 10,000 g, and the supernatant was collected and used for measuring TNF-α concentration (pg/mL), IL-1β concentration (pg/mL), IL-6 concentration (pg/mL), and total protein concentration (µg/mL), respectively. An ELISA assay kit was used for the TNF-α, IL-1β, and IL-6 concentrations. The total protein concentration was measured by using a Pierce (trademark) BCA Protein Assay Kit. The inflammatory cytokine level (pg/mg protein) in the total protein was calculated from the TNF-α concentration (pg/mL), IL-1β concentration (pg/mL), and IL-6 concentration (pg/mL), and total protein concentration (µg/mL).

The results are described in Table 11.

### (Example 2: Test using model animals of diabetic nephropathy)

### <Preparing diabetic nephropathy model animals>

Male db/db mice (16 weeks old) were used as model mice of type 2 diabetes. To induce diabetic nephropathy early, at 6 weeks old, an approximately 2 cm incision was made in the right abdomen under isoflurane anesthesia, the renal arteriovenous and ureteral stent were ligated, and then the right kidney was excised to produce diabetic nephropathy model mice. Male C57BL/6J mice (16 weeks old) were used as a control group.

### «Administering drugs»

Six per group of the diabetic nephropathy model mice prepared above were randomly assigned to each of a DN group, an STMP-27 (30 mg/kg) group, an SMTP-44D (30 mg/kg) group, and a metformin group. Moreover, six of the above-mentioned C57BL/6J mice were assigned as a control group.

The SMT P-27 (30 mg/kg) group and SMTP-44D (30 mg/kg) group were intraperitoneally (i.p.) administered SMT P-27 (30 mg/kg dose) or SMTP-44D (30 mg/kg dose) once every two days for 10 continuous weeks from 6 weeks old to 16 weeks old.

The DN group and control group were intraperitoneally (i.p.) administered physiological saline once every two days for 10 continuous weeks from 6 weeks old to 16 weeks old.

The metformin group was orally administered metformin (300 mg /kg) once every two days for 10 continuous weeks from 6 weeks old to 16 weeks old.

Body weight (BW), blood glucose level (BS), urine volume (UF), urinary albumin (Ualb), serum creatinine (Scr), creatinine clearance (Ccr), and tubular regeneration score were measured at 16 weeks old for the above-mentioned DN group, STMP-27 group, SMTP-44D groups, metformin group, and control group.

### <Evaluations>

### [Measuring body weight and blood glucose level]

Body weight and blood glucose level were measured similarly to the above-mentioned example 1.

The measurement results are described in Table 12.

### [Measuring urine volume]

Urine was collected for 24 hours at 16 weeks old for the above-mentioned DN group, STMP-27 group, SMTP-44D groups, metformin group, and control group mice using a metabolic cage, and urine volume (UF: µL) was measured. During the urine collection, only free drinking of water was allowed.

The measurement results are described in Table 12.

### [Measuring urinary albumin, serum creatinine, and creatinine clearance]

After the urine collection, blood were collected from the abdominal aortas of the above-mentioned DN group, STMP-27 group, SMTP-44D groups, metformin group, and control group mice at 16 weeks old under isoflurane anesthesia. The collected blood was centrifuged at 25°C for 15 minutes at 800 g, and the supernatants were collected. Moreover, the urine collected for measuring the above-mentioned urine volume was used to evaluate the following renal functions. To evaluate the renal functions, serum creatinine concentration (Scr; mg/dL) and urine creatinine concentration (Ucr; mg/dL) were measured using a LabAssay (trademark) Creatinine, and thus the creatinine clearance (Ccr; mL/min) was calculated. The urine albumin concentration (Ualb; mg/hr/kg) was measured using an Albuwell M.

The measurement results are described in Table 12.

### [Measuring tubular regeneration score]

After the blood collection, the left kidney was excised from the above-mentioned DN group, STMP-27 group, SMTP-44D groups, metformin group, and control group mice at 16 weeks old, and the fat was ablated in physiological saline. To conduct hematoxylin eosin (H E) dyeing, the kidneys were fixed in 10% neutral buffered formalin liquid. Using an automatic embedding device, the kidneys fixed in formalin were penetrated with paraffin to form paraffin blocks. The paraffin blocks were thinly cut into 3-µm segments, which were put on slide glasses. The segments were expanded using a paraffin expander at 52°C and dried sufficiently using a paraffin melter at 37°C for one night. The segments were deparaffinized and washed in water, dyed with Mayer's hematoxylin staining solution for one minute, and washed in running water for 15 minutes. Then, the segments were dyed using an eosin solution for 45 minutes, dehydrated using 80 to 100% ethanol, cleared using xylene, and sealed using Malinol. The specimens for the respective groups were examined using an optical microscope at 100× magnification. By grading the regenerated renal tubules as 0 points for no lesions, 1 point for a slight change, 2 points for a mild change, 3 points for a moderate change, and 4s point for a severe change, tubular regeneration scores (points) were measured.

The measurement results are described in Table 12.

**[Table 9]**

| **BW(g)** | | | | | |
|---|---|---|---|---|---|
| Group | 0th week | 1st week | **2nd week** | **3rd week** | **4th week** |
| **Control** | 22.8 ± 02 | 22.8 ± 0.1 | 23.3 ± 0.1 | 23.75 ± 0.2 | 24.3 ± 0.2 |
| STZ | 23.7 ± 0.3 | 21.8 ± 0.3^{∗} | 21.25 ± 0 4 ^{∗} | 20.8 ± 0.6 ^{∗∗} | 20 1 ± 0 5^{∗∗} |
| **SMTP-44D (30 mg/kg)** | 23.0 ± 0.4 | 21.7 ± 0.5 ^{∗} | 2L6 ± 0.5 ^{∗} | 21.3 ± 0.6 ^{∗∗} | 21.5 ± 0.5 ^{∗∗} |
| **Edaravone (10 mg/kg)** | 23.4 ± 0.3 | 21.9 ± 0.4 | 21 6 ± 04^{∗} | 21.3 ± 0.5 ^{∗∗} | 21.0 ± 0.5 ^{∗∗} |
| **Pregabalin (10 mg/kg)** | 21.8 ± 0.3 | 21.4 ± 0.3^{∗∗} | 21.3 ± 0.4 ^{∗} | 21.0 ± 02^{∗∗} | 20.4 ± 0.2 ^{∗∗} |

| **BS (mg/dL)** | | | | | |
|---|---|---|---|---|---|
| Group | 0th week | 1st week | **2nd week** | **3rd week** | **4th week** |
| **Control** | 186.3 ± 8.3 | 200.0 ± 6.7 | 173.8 ± 6.1 | 177.7 ± 9.9 | 165.0 ± 8.0 |
| **STZ** | 179.5 ± 11.7 | 507.0 ± 27J ^{∗∗} | 568.2 ± 24.7 ^{∗∗} | 668.8 ± 31.6 ^{∗∗} | 596.4 ± 24.6 ^{∗∗} |
| **SMTP-4D (30 mg/kg)** | 203.5 ± 5.5 | 535 5 ± 28.7 ^{∗∗} | 597.3 ± 448 ^{∗∗} | 638.7 ± 34.2 ^{∗∗} | 580.3 ± 42.9 ^{∗∗} |
| **Edaravone (10 mg/kg)** | 221.0 ± 73 | 566.0 ± 32.6 ^{∗∗} | 567.5 ± 28 0 ^{∗∗} | 617.7 ± 373.3 | 565.3 ± 35.1 ^{∗∗} |
| **Pregabalin (10 mg/kg)** | 192.2 ± 5.3 | 562 7 ± 25.6 ^{∗∗} | 639.1 ± 44.7 ^{∗∗} | 657.2 ± 38.7 ^{∗∗} | 582.3 ± 38.2 ^{∗∗} |

**[Table 10]**

| von Frey test (g) | | | | | |
|---|---|---|---|---|---|
| Group | 0th week | 1st week | 2nd week | 3rd week | 4th week |
| Control | 4.8 ± 0 2 | 4.7 ± 0.3 | 4.7 ± 02 | 4.9 ± 0.2 | 4.6 ± 0 2 |
| STZ | 4.8 ± 0 2 | 4.1 ± 0.2 ^{∗∗} | 3.3 ± 0.2 ^{∗∗} | 3.4 ± 02 ^{∗∗} | 3.4 ± 0.2 ^{∗∗} |
| SMTP-44D (0.3 mg/kg) | 5.2 ± 0.1 | 4.1 ± 0.1 ^{∗∗} | 3 5 ± 0 2 | 3.7 ± 0.1 | 3.6 ± 0.2 |
| SMTP-44D (3 mg/kg) | 4.9 ± 0.2 | 4.2 ± 0 1 ^{∗∗} | 4.0 ± 0 1 ##- | 3.9 ± 0.2 # | 4.0 ± 0.1 ## |
| SMTP-44D (30 mg/kg) | 4.9 ± 0 1 | 3.9 ± 0.2 ^{∗} | 5 4 ± 0.1 ## | 5.3 ± 0.1 ## | 5.2 ± 0 1 ## |
| Edaravone (10 mg/kg) | 5 1 ± 0 1 | 4.1 ± 0.1 ^{∗∗} | 4.6 ± 0.2 ## | 4.8 ± 0.1 ## | 5.1 ± 0.2 ## |
| Pregabalin (10 mg/kg) | 5.0 ± 02 | 4.0 ± 0.1 ^{∗∗} | 4.6 ± 0.2 ## | 4.6 ± 0.2 ## | 4 7 ± 02 ## |
| | | | | | |

| Hot plate test (sec) | | | | | |
|---|---|---|---|---|---|
| Group | 0th week | 1st week | 2nd week | 3rd week | 4th week |
| Control | 20. ± 0.8 | 21.2 ± 0.8 | 19.6 ± 1.0 | 20.0 ± 0.9 | 20.4 ± 0.9 |
| STZ | 20.6 ± 1.0 | 16.4 ± 0.9 ^{∗∗} | 14.1 ± 1.3 ^{∗∗} | 12 6 ± 0.8 ^{∗∗} | 13.0 ± 1.2 ^{∗∗} |
| SMTP-44D (0.3 mg/kg) | 22.8 ± 1.2 | 14.3 ± 1.0 ^{∗∗} | 14.0 ± 1.4 | 13.3 ± 1.3 | 13.1 ± 0.6 |
| SMTP-44D (3 mg/kg) | 21.0 ± 1.3 | 14.6 ± 1.4 ^{∗∗} | 15.4 ± 0.8 | 15.1 ± 1.1 | 15.1 ± 1.3 |
| SMTP-44D (30 mg/kg) | 21.3 ± 0.4 | 15.0 ± 0.7 ^{∗∗} | 18.0 ± 1.2 ## | 18.4 ± 1.3 ## | 18 2 ± 1.1 ## |
| Edaravone (10 mg/kg) | 20.8 ± 0.5 | 15.7 ± 0.8 ^{∗∗} | 15.5 ± 1.2 | 15.4 ± 0.9 | 16.0 ± 0.8 # |
| Pregabalin (10 mg/kg) | 22.2 ± 0.8 | 15.7 ± 0.7 ^{∗∗} | 17.3 ± 1.1 # | 17.7 ± 0.9 ## | 17.9 ± 1.1 ## |
| | | | | | |

| Hind plantar blood flow (PU): | | | | | |
|---|---|---|---|---|---|
| Group | 0th week | 1st week | 2nd week | 3rd week | 4th week |
| Control | 186.8 ± 18.8 | 213.1 ± 16.1 | 200.2 ± 9.7 | 197.5 ± 17.6 | 188.5 ± 86 |
| STZ | 230.9 ± 13.6 | 128.9 ± 10.6 ^{∗∗} | 141.7 ± 7.2 ^{∗∗} | 130.7 ± 10.2 ^{∗∗} | 122.5 ± 7.3 ^{∗∗} |
| SMTP-44D (0.3 mg/kg) | 199.8 ± 9.8 | 150.7 ± 9.4 ^{∗∗} | 152.9 ± 8.7 | 141.0 ± 8.7 | 132.3 ± 6.6 |
| SMTP-44D (3 mg/kg) | 210.7 ± 14.1 | 154.8 ± 12.8 ^{∗∗} | 1693 ± 7.9 ## | 161.3 ± 11.8 # | 162.1 ± 9.8 ## |
| SMTP-44D (30 mg/kg) | 201.4 ± 6.8 | 142.7 ± 7.1 ^{∗∗} | 185.0 ± 8.4 ## | 234.7 ± 11.2 ## | 214.9 ± 12 2 ## |
| Edaravone (10 mg/kg) | 227.2 ± 12.5 | 149.7 ± 11.5 ^{∗∗} | 16L4 ± 9.1 # | 166.6 ± 7.5 ## | 178.2 ± 9.9 ## |
| Pregabalin (10 mg/kg) | 214.2 ± 7.6 | 159.8 ± 6.3 ^{∗∗} | 144.7 ± 8.3 | 152.7 ± 9.5 | 151.2 ± 8.0 ## |

**[Table 11]**

| Group/4th week | | | Sciatic nerve blood flow (PU) | | | Sciatic nerve conduction velocity (m/s) | |
|---|---|---|---|---|---|---|---|
| Control | | | 1481.9 ± 55.0 | | | 74.6 ± 2.2 | |
| STZ | | | 1133.2 ± 66.8 ^{∗∗} | | | 54.9 ± 2.4 ^{∗∗} | |
| SMTP-44D (0.3 mg/kg) | | | 1116.9 ± 30.4 | | | 54.9 ± 1.2 | |
| SMTP-44D (3 mg/kg) | | | 1187.1 ± 31.4 | | | 56.9 ± 2.8 | |
| SMTP-44D (30 mg/kg) | | | 1461.0 ± 55.0 ## | | | 63.8 ± 2.0 ## | |
| Edaravone (10 mg/kg) | | | 1336.1 ± 46.7 ## | | | 63.6 ± 1.8 ## | |
| Pregabalin (10 mg/kg) | | | 1114.1 ± 27.5 | | | 55.9 ± 1.4 | |
| | | | | | | | |

| Group/4th week | | Thickness of myelin (µm) | | | G-ratio | | |
|---|---|---|---|---|---|---|---|
| Control | | 1.47 ± 0.04 | | | 0.556 ± 0.008 | | |
| STZ | | 1.24 ± 0.02 ^{∗∗} | | | 0.657 ± 0.004 ^{∗∗} | | |
| SMTP-44D (30 mg/kg) | | 1.58 ± 0.02 ## | | | 0.585 ± 0.004 ## | | |
| Edaravone (10 mg/kg) | | 1.42 ± 0.02 ## | | | 0.619 ± 0.005 ## | | |
| Pregabalin (10 mg/kg) | | 1.29 ± 0.03 | | | 0.665 ± 0.006 | | |
| | | | | | | | |

| Group/4th week | MDA (nmol/mg protein) | | | TNF-α (pg/mg protein) | | IL-1β (pg/mg protein) | IL-6 (pg/mg protein) |
|---|---|---|---|---|---|---|---|
| Control | 1.65 ± 0.41 | | | 9.68 ± 0.85 | | 11.89 ± 2.01 | 121.43 ± 5.04 |
| STZ | 4.86 ± 1.37 ^{∗} | | | 26.35 ± 3.01 ^{∗∗} | | 48.42 ± 8.12 ^{∗∗} | 228.39 ± 16.83 ^{∗∗} |
| SMTP-44D (30 mg/kg) | 1.98 ± 0.51 ## | | | 15.22 ± 2.51 ## | | 24.90 ± 4.05 ## | 143.41 ± 8.08 ## |
| Edaravone (10 mg/kg) | 2.98 ± 0.39 | | | 12.45 ± 2.06 ## | | 24.54 ± 4.52 ## | 165.83 ± 10.21 ## |
| Pregabalin (10 mg/kg) | 4.02 ± 0.59 | | | 14.53 ± 1.41 ## | | 58.04 ± 4.47 | 166.60 ± 13.57 ## |

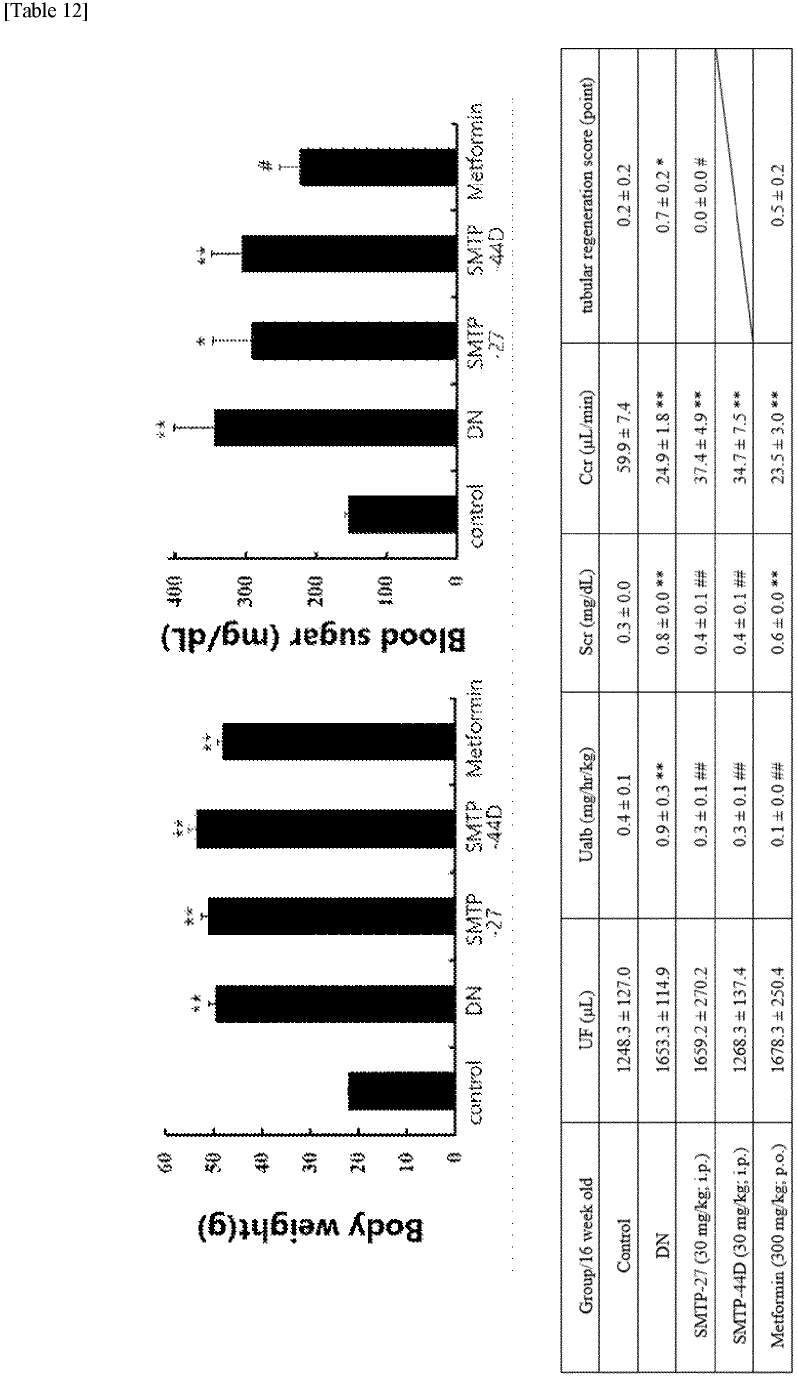

In Tables 9 to 12, the experimental results are expressed as the mean value ± standard deviation. As comparisons among multiple groups, first a one-way analysis of variance (ANOVA) was conducted, and a Bonferroni test was conducted for those showing significant differences. Note, to evaluate the regenerated renal tubules, a Steel-Dwass test was conducted. Those with a significance level lower than 5% (P<0.05) were determined as having significant differences.

In Tables 9 to 12, "^{∗}" expresses P<0.05 with respect to the control group, and "^{∗∗}" expresses P<0.01 with respect to the control group.

In Tables 9 to 12, "#" expresses P<0.05 with respect to the STZ group or DN group, and "##" expresses P<0.01 with respect to the STZ group or DN group.

In FIG. 12, the location with a diagonal (SMTP-44D group tubular regeneration score) expresses that no data were obtained.

### <Results of Example 1>

### [Changes in body weights (BW) when SMTP-44D, edaravone, or pregabalin was administered]

The body weights for the control group (0th week: 22.8±0.2 g; 1st week: 22.8±0.1 g; 2nd week: 23.3±0.1 g; 3rd week: 23.75±0.2 g; 4th week: 24.3±0.2 g) increased by 1.5 g between the 0th week and 4th week. Meanwhile, the body weights for the STZ group (0th week: 23.7±0.3 g; 1st week: 21.8±0.3 g; 2nd week: 21.25±0.4 g; 3rd week: 20.8±0.6 g; 4th week: 20.1±0.5 g) decreased by 3.6 g between the 0th week and 4th week.

The body weights for the SMTP-44D (30 mg/kg) group (0th week: 23.0±0.4 g; 1st week: 21.7±0.5 g; 2nd week: 21.6±0.5 g; 3rd week: 21.3±0.6 g; 4th week: 21.5±0.5 g) decreased by 1.5 g between the 0th week and 4th week. Moreover, the body weights for the edaravone (10 mg/kg) group (0th week: 23.4±0.3 g; 1st week: 21.9±0.4 g; 2nd week: 21.6±0.4 g; 3rd week: 21.3±0.5 g; 4th week: 21.0±0.5 g) decreased by 2.4 g between the 0th week and 4th week. The body weights for the pregabalin (10 mg/kg) group (0th week: 21.8±0.3 g; 1st week: 21.4±0.3 g; 2nd week: 21.3±0.4 g; 3rd week: 21.0±0.2 g; 4th week: 20.4±0.2 g) decreased by 1.4 g between the 0th week and 4th week.

### [Changes in blood glucose levels (BS) following SMTP-44D, edaravone, or pregabalin administration]

Blood glucose levels in the STZ group (week 0: 179.5 ± 11.7 mg/dL, week 1: 507.0 ± 27.1 mg/dL, week 2: 568.2 ± 24.7 mg/dL, week 3: 668.8 ± 31.6 mg/dL, week 4: 596.4 ± 24.6 mg/dL) were observed to have increased significantly starting from week 1 compared to blood glucose levels in the control group (week 0: 186.3 ± 8.3 mg/dL, week 1: 200.0 ± 6.7 mg/dL, week 2: 173.8 ± 6.1 mg/dL, week 3: 177.7 ± 9.9 mg/dL, week 4: 165.0 ± 8.0 mg/dL).

Blood glucose levels in the SMTP-44D (30 mg/kg) group (week 0: 203.5 ± 5.5 mg/dL, week 1: 535.5 ± 28.7 mg/dL, week 2: 597.3 ± 44.8 mg/dL, week 3: 638.7 ± 34.2 mg/dL, week 4: 580.3 ± 42.9 mg/dL), blood glucose levels in the edaravone (10 mg/kg) group (week 0: 221.0 ± 7.3 mg/dL, week 1: 566.0 ± 32.6 mg/dL, week 2: 567.5 ± 28.0 mg/dL, week 3: 617.7 ± 37.3 mg/dL, week 4: 565.3 ± 35.1 mg/dL), and blood glucose levels in the pregabalin (10mg/kg) group (week 0: 192.2 ± 5.3 mg/dL, week 1: 562.7 ± 25.6 mg/dL, week 2: 639.1 ± 44.7 mg/dL, week 3: 657.2 ± 38.7 mg/dL, week 4: 582.3 ± 38.2 mg/dL) were observed to have no significant changes compared to the STZ group.

### [Changes in mechanical threshold value using a von Frey test following SMTP-44D, edaravone, or pregabalin administration]

Mechanical threshold values in the STZ group (week 0: 4.8 ± 0.2 g, week 1: 4.1 ± 0.2 g, week 2: 3.3 ± 0.2 g, week 3: 3.4 ± 0.2 g, week 4: 3.4 ± 0.2 g) fell significantly starting from week 1 compared to mechanical threshold values in the control group (week 0: 4.8 ± 0.2 g, week 1: 4.7 ± 0.3 g, week 2: 4.7 ± 0.2 g, week 3: 4.9 ± 0.2 g, week 4: 4.6 ± 0.2 g).

Mechanical threshold values in the SMTP-44D (3 mg/kg) group (week 0: 4.9 ± 0.2 g, week 1: 4.2 ± 0.1 g, week 2: 4.0 ± 0.1 g, week 3: 3.9 ± 0.2 g, week 4: 4.0 ± 0.1 g), mechanical threshold values in the SMTP-44D (30 mg/kg) group (week 0: 4.9 ± 0.1 g, week 1: 3.9 ± 0.2 g, week 2: 5.4 ± 0.1 g, week 3: 5.3 ± 0.1 g, week 4: 5.2 ± 0.1 g), mechanical threshold values in the edaravone (10 mg/kg) group (week 0: 5.1 ± 0.1 g, week 1: 4.1 ± 0.1 g, week 2: 4.6 ± 0.2 g, week 3: 4.8 ± 0.1 g, week 4: 5.1 ± 0.2 g), and mechanical threshold values in the pregabalin (10 mg/kg) group (week 0: 5.0 ± 0.2 g, week 1: 4.0 ± 0.1 g, week 2: 4.6 ± 0.2 g, week 3: 4.6 ± 0.2 g, week 4: 4.7 ± 0.2 g) were observed to show significant improvements starting in the second week compared to the STZ group.

### [Changes in heat threshold values using a hot plate test following SMTP-44D, edaravone, or pregabalin administration]

Heat threshold values for the STZ group (week 0: 20.6 ± 1.0 s, week 1: 16.4 ± 0.9 s, week 2: 14.1 ± 1.3 s, week 3: 12.6 ± 0.8 s, week 4: 13.0 ± 1.2 s) dropped significantly starting in week 1 compared to the heat threshold values in the control group (week 0: 20.8 ± 0.8 s, week 1: 21.2 ± 0.8 s, week 2: 19.6 ± 1.0 s, week 3: 20.0 ± 0.9 s, week 4: 20.4 ± 0.9 s).

No significant improvements were observed in the heat threshold values in the SMTP-44D (0.3 mg/kg) group (week 0: 22.8 ± 1.2 s, week 1: 14.3 ± 1.0 s, week 2: 14.0 ± 1.4 s, week 3: 13.3 ± 1.3 s, week 4: 13.1 ± 0.6 s) or heat threshold values in the SMTP-44D (3 mg/kg) group (week 0: 21.0 ± 1.3 s, week 1: 14.6 ± 1.4 s, week 2: 15.4 ± 0.8 s, week 3: 15.1 ± 1.1 s, week 4: 15.1 ± 1.3 s) compared to the STZ group.

Significant improvements were observed starting in week 2 in the heat threshold values for the SMTP-44D (30 mg/kg) group (week 0: 21.3 ± 0.4 s, week 1: 15.0 ± 0.7 s, week 2: 18.0 ± 1.2 s, week 3: 18.4 ± 1.3 s, week 4: 18.2 ± 1.1 s) and the heat threshold values for the pregabalin (10 mg/kg) group (week 0: 22.2 ± 0.8 s, week 1: 15.7 ± 0.7 s, week 2: 17.3 ± 1.1 s, week 3: 17.7 ± 0.9 s, week 4: 17.9 ± 1.1 s) compared to the STZ group.

Significant improvements were observed starting in week 4 in the heat threshold values of the edaravone (10 mg/kg) group (week 0: 20.8 ± 0.5 s, week 1: 15.7 ± 0.8 s, week 2: 15.5 ± 1.2 s, week 3: 15.4 ± 0.9 s, week 4: 16.0 ± 0.8 s) compared to the STZ group.

### [Changes in hind plantar blood flow following SMTP-44D, edaravone, or pregabalin administration]

Hind plantar blood flow in the STZ group (week 0: 230.9 ± 13.6 PU, week 1: 128.9 ± 10.6 PU, week 2: 141.7 ± 7.2 PU, week 3: 130.7 ± 10.2 PU, week 4: 122.5 ± 7.3 PU) fell significantly starting in week 1 compared to the hind plantar blood flow in the control group (week 0: 186.8 ± 18.8 PU, week 1: 213.1 ± 16.1 PU, week 2: 200.2 ± 9.7 PU, week 3: 197.5 ± 17.6 PU, week 4: 188.5 ± 8.6 PU).

No significant improvements were observed in the hind plantar blood flow in the SMTP-44D (0.3 mg/kg) group (week 0: 199.8 ± 9.8 PU, week 1: 150.7 ± 9.4 PU, week 2: 152.9 ± 8.7 PU, week 3: 141.0 ± 8.7 PU, week 4: 132.3 ± 6.6 PU) compared to the STZ group.

Significant improvements were observed starting in week 2 in the hind plantar blood flow in the SMTP-44D (3 mg/kg) group (week 0: 210.7 ± 14.1 PU, week 1: 154.8 ± 12.8 PU, week 2: 169.3 ± 7.9 PU, week 3: 161.3 ± 11.8 PU, week 4: 162.1 ± 9.8 PU), the hind plantar blood flow in the SMTP-44D (30 mg/kg) group (week 0: 201.4 ± 6.8 PU, week 1: 142.7 ± 7.1 PU, week 2: 185.0 ± 8.4 PU, week 3: 234.7 ± 11.2 PU, week 4: 214.9 ± 12.2 PU), and the hind plantar blood flow in the edaravone (10 mg/kg) group (week 0: 227.2 ± 12.5 PU, week 1: 149.7 ± 11.5 PU, week 2: 161.4 ± 9.1 PU, week 3: 166.6 ± 7.5 PU, week 4: 178.2 ± 9.9 PU) compared to the STZ group.

Significant improvements were observed starting in week 4 in the hind plantar blood flow in the pregabalin (10 mg/kg) group (week 0: 214.2 ± 7.6 PU, week 1: 159.8 ± 6.3 PU, week 2: 144.7 ± 8.3 PU, week 3: 152.7 ± 9.5 PU, week 4: 151.2 ± 8.0 PU) compared to the STZ group.

### [Changes in sciatic nerve blood flow following SMTP-44D, edaravone, or pregabalin administration]

It was observed that the sciatic nerve blood flow in the STZ group (1133.2 ± 66.8 PU) fell significantly compared to the sciatic nerve blood flow in the control group (1481.9 ± 53.0 PU).

No significant improvements were observed in the sciatic nerve blood flow in the SMTP-44D (0.3 mg/kg) group (1116.9 ± 30.4 PU), the sciatic nerve blood flow in the SMTP-44D (3 mg/kg) group (1187.1 ± 31.4 PU), or the sciatic nerve blood flow in the pregabalin (10 mg/kg) group (1114.1 ± 27.5 PU) compared to the STZ group.

Significant improvements were observed in the sciatic nerve blood flow in the SMTP-44D (30 mg/kg) group (1461.0 ± 55.0 PU) and in the sciatic nerve blood flow in the edaravone (10 mg/kg) group (1336.1 ± 46.7 PU) compared to the STZ group.

### [Changes in sciatic nerve conduction velocity following SMTP-44D, edaravone, or pregabalin administration]

A significant decrease was observed in the sciatic nerve conduction velocity of the STZ group (54.9 ± 2.4 m/s) compared to the sciatic nerve conduction velocity in the control group (74.6 ± 2.2 m/s).

No significant improvements were observed in the sciatic nerve conduction velocity of the SMTP-44D (0.3 mg/kg) group (54.9 ± 1.2 m/s), the sciatic nerve conduction velocity in the SMTP-44D (3 mg/kg) group (56.9 ± 2.8 m/s), or the sciatic nerve conduction velocity in the pregabalin (10 mg/kg) group (55.9 ± 1.4 m/s) compared to the STZ group.

Significant improvements were observed in the sciatic nerve conduction velocity in the SMTP-44D (30 mg/kg) group (63.8 ± 2.0 m/s) and the sciatic nerve conduction velocity in the edaravone (10 mg/kg) group (63.6 ± 1.8 m/s) compared to the STZ group.

### [Changes in myelin thickness and G-ratio following SMTP-44D, edaravone, or pregabalin administration]

A significant decrease was observed in myelin thickness in the STZ group (1.24 ± 0.02 µm) compared to the myelin thickness in the control group (1.47 ± 0.04 µm).

Significant improvements were observed in the myelin thickness in the SMTP-44D (30 mg/kg) group (1.58 ± 0.02 µm) and the myelin thickness in the edaravone (10 mg/kg) group (1.42 ± 0.02 µm) compared to the STZ group.

No significant improvements were observed in the myelin thickness in the pregabalin (10 mg/kg) group (1.29 ± 0.03 µm) compared to the STZ group.

A significant increase was observed in the G-ratio of the STZ group (0.657 ± 0.004) compared to the G-ratio in the control group (0.556 ± 0.008).

Significant improvements were observed in the G-ratio of the SMTP-44D (30 mg/kg) group (0.585 ± 0.004) and the G-ratio in the edaravone (10 mg/kg) group (0.619 ± 0.005) compared to the STZ group.

No significant improvements were observed in the G-ratio of the pregabalin (10 mg/kg) group (0.665 ± 0.006) compared to the STZ group.

### [Changes in oxidative stress following SMTP-44D, edaravone, or pregabalin administration]

A significant increase was observed in oxidative stress in the STZ group (4.86 ± 1.37 nmol/mg protein) compared to oxidative stress in the control group (1.65 ± 0.41 nmol/mg protein).

Significant improvements were observed in oxidative stress in the SMTP-44D (30 mg/kg) group (1.98 ± 0.51 nmol/mg protein) compared to the STZ group.

No significant improvements were observed in oxidative stress in the edaravone (10 mg/kg) group (2.98 ± 0.39 nmol/mg protein) and the pregabalin (10 mg/kg) group (4.02 ± 0.59 nmol/mg protein) compared to the STZ group.

### [Changes in inflammatory cytokines following SMTP-44D, edaravone, or pregabalin administration]

A significant increase was observed in the TNF-α (26.35 ± 3.01 pg/mg protein) and IL-6 (228.39 ± 16.83 pg/mg protein) in the STZ group compared to the TNF-α (9.68 ± 0.85 pg/mg protein) and IL-6 (121.43 ± 5.04 pg/mg protein) in the control group.

Significant improvements were observed in the TNF-α (15.22 ± 2.51 pg/mg protein) and IL-6 (143.41 ± 8.08 pg/mg protein) in the SMTP-44D (30 mg/kg) group, the TNF-α (12.45 ± 2.06 pg/mg protein) and IL-6 (165.83 ± 10.21 pg/mg protein) in the edaravone (10 mg/kg) group, and the TNF-α (14.53 ± 1.41 pg/mg protein) and IL-6 (166.60 ± 13.57 pg/mg protein) in the pregabalin (10 mg/kg) group compared to the STZ group.

A significant increase was observed in the IL-1β (48.42 ± 8.12 pg/mg protein) in the STZ group compared to the IL-1β (11.89 ± 2.01 pg/mg protein) in the control group.

Significant improvements were observed in the IL-1β in the SMTP-44D (30 mg/kg) group (24.90 ± 4.05 pg/mg protein) and the IL-1β in the edaravone (10 mg/kg) group (24.54 ± 4.52 pg/mg protein) compared to the STZ group.

No significant improvements were observed in the IL-1β of the pregabalin (10 mg/kg) group (58.04 ± 4.47 pg/mg protein) compared to the STZ group.

### <Results of embodiment 2>

### [Changes in body weight following administration of SMTP-27, SMTP-44D, or metformin]

No significant changes were observed in body weight in the SMTP-27 (30 mg/kg) group and the SMTP-44D (30 mg/kg) group compared to the DN group.

### [Changes in blood glucose levels following administration of SMTP-27, SMTP-44D, or metformin]

No significant changes were observed in blood glucose levels in the SMTP-27 (30 mg/kg) group and the SMTP-44D (30 mg/kg) group compared to the DN group.

### [Changes in urine volume following administration of SMTP-27, SMTP-44D, or metformin]

No significant changes were observed in urine volume in the DN group (1653.3 ± 114.9 µL) compared to the urine volume in the control group (1248.3 ± 127.9 µL).

No significant improvements were observed in the urine volume of the SMTP-27 (30 mg/kg) group (1659.2 ± 270.2 µL), the urine volume in the SMTP-44D (30 mg/kg) group (1268.3 ± 137.4 µL), or the urine volume in the metformin (300 mg/kg) group (1678.3 ± 250.4 µL) compared to the DN group.

### [Changes in urinary albumin, serum creatinine, and creatinine clearance following administration of SMTP-27, SMTP-44D, or metformin]

A significant increase was observed in the urinary albumin in the DN group (0.9 ± 0.3 mg/hr/kg) compared to the urinary albumin in the control group (0.4 ± 0.1 mg/hr/kg).

Significant improvements were observed in the urinary albumin in the SMTP-27 (30 mg/kg) group (0.3 ± 0.1 mg/hr/kg), the urinary albumin in the SMTP-44D (30 mg/kg) group (0.3 ± 0.1 mg/hr/kg), and the urinary albumin in the metformin (300 mg/kg) group (0.1 ± 0.0 mg/hr/kg) compared to the DN group.

A significant increase was observed in the serum creatinine in the DN group (0.8 ± 0.0 mg/dL) compared to the serum creatinine in the control group (0.3 ± 0.0 mg/dL).

Significant improvements were observed in the serum creatinine in the SMTP-27 (30 mg/kg) group (0.4 ± 0.1 mg/dL) and the SMTP-44D (30 mg/kg) group (0.4 ± 0.1 mg/dL) compared to the DN group.

No significant improvements were observed in the serum creatinine in the metformin (300 mg/kg) group (0.6 ± 0.0 mg/dL) compared to the DN group.

A significant drop was observed in the creatinine clearance (24.9 ± 1.8 µL/min) in the DN group compared to the creatinine clearance in the control group (59.9 ± 7.4 µL/min).

No significant improvements were observed in the creatinine clearance in the SMTP-27 (30 mg/kg) group (37.4 ± 4.9 µL/min), the creatinine clearance in the SMTP-44D (30 mg/kg) group (34.7 ± 7.5 µL/min), and the creatinine clearance in the metformin (300 mg/kg) group (23.5 ± 3.0 nL/min) compared to the DN group.

A significant increase was observed in tubular regeneration score (0.7 ± 0.2 pts) in the DN group compared to the tubular regeneration score (0.2 ± 0.2 pts) in the control group.

A significant improvement was observed in the tubular regeneration score in the SMTP-27 (30 mg/kg) group (0.0 ± 0.0 pts) compared to the DN group.

A significant improvement was observed in the tubular regeneration score in the metformin (300 mg/kg) group (0.5 ± 0.2 pts) compared to the DN group.

As shown in Tables 9 to 12, the SMTP-44D groups displayed a dose-dependent improvement effect on diabetic neuropathy equal to or greater than that of the edaravone group and the pregabalin group. Furthermore, the SMTP-27 and SMTP-44D groups displayed an improvement effect with respect to diabetic neuropathy equal to or greater than that of the metformin group. These results show that a drug containing the compound represented by formula (I) of the present disclosure has therapeutic and prophylactic effects on diabetes complications such as diabetic neuropathy and diabetic kidney disease.

The disclosure of Japanese Patent Application No. 2019-044670 filed on March 12, 2019 is incorporated herein in its entirety by reference.

All documents, patent applications, and technical standards described herein are incorporated herein by reference to the same extent as if incorporation by reference of the individual documents, patent applications, and technical standards were stated specifically and individually.

## Claims

1. A drug used for diabetes complications, comprising a compound represented by formula (I) below as an active ingredient: 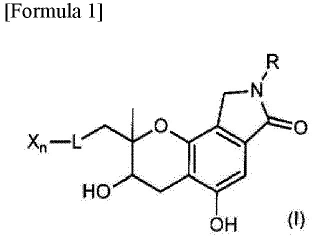 wherein, in formula (I), L represents a 4 to 10 carbon aliphatic hydrocarbon group, X represents a hydroxy group or a carboxy group, n represents an integer of 0 to 2, and R represents a hydrogen atom or a substituent having a molecular weight of 1,000 or less.

2. The drug according to claim 1, wherein the compound represented by the formula (I) is a compound represented by formula (IA) below: 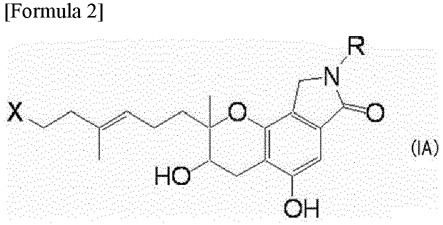 and in formula (IA), X is -CHY-C(CH₃)₂Z, Y and Z are each independently -H or -OH or form a single bond together, and R represents a hydrogen atom or a substituent having a molecular weight of 1,000 or less.

3. The drug according to claim 1 or claim 2, wherein the compound represented by the formula (I) is a compound represented by formula (II) or formula (III) below: 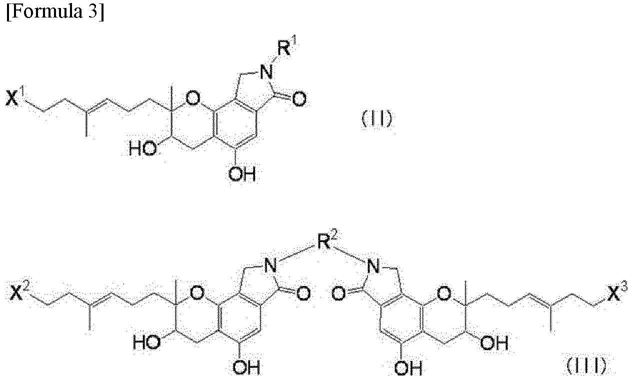 in formula (II) or formula (III), X¹, X², and X³ are each independently -CHY-C(CH₃)₂Z, Y and Z are each independently -H or -OH or form a single bond together, and R¹ represents any one of the following (A) to (D):
(A) a residue obtained by removing a single amino group from an amino compound selected from the group consisting of natural amino acids, D isomers of natural amino acids, and compounds obtained by substituting at least one carboxyl group with a hydrogen atom, a hydroxy group, or a hydroxymethyl group in natural amino acids and D isomers of natural amino acids (however, this excludes -(CH)₂-OH)
(B) an aromatic group having at least one selected from the group consisting of a carboxyl group, a hydroxyl group, a sulfonic acid group, and a secondary amino group as a substituent or as a part of a substituent, or an aromatic group containing a secondary amino group and optionally containing a nitrogen atom
(C) an aromatic amino acid residue represented by formula (II-1) (in the formula, R³ are each independently and optionally a substituent, and if present, represent a hydroxy group, a carboxyl group, or a 1 to 5 carbon alkyl group, n represents an integer of 0 or 1, m represents an integer of 0 to 5, and ^{∗} represents a binding site) 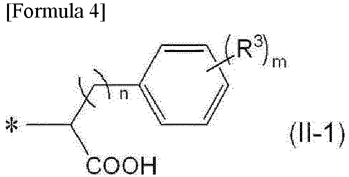
(D) a substituent represented by -L¹-L²-R⁴- (in the formula, L¹ represents a linking group which is a 1 to 4 carbon alkene group having a carboxy group, L² represents a linking group represented by -NH-C(=O)- or -NH-C(=S)-NH-, and R⁴ is a 9-fluorenylalkoxy group having a 1 to 3 carbon alkoxy group or a poly heterocyclic group represented by formula (II-2) below (in the formula (II-2), ^{∗} represents a binding site): 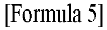 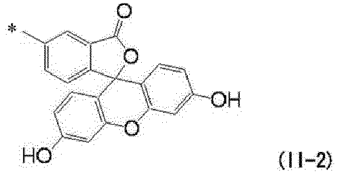 and R² represents a residue obtained by removing two amino groups from an amino compound selected from the groups consisting of: natural amino acids having two amino groups, D isomers of natural amino acids having two amino groups, compounds in which at least one carboxyl group in a natural amino acid having two amino groups or a D isomer of a natural amino acid having two amino groups is replaced with a hydrogen atom, a hydroxyl or a hydroxymethyl group, and compounds represented by H₂N-CH(COOH)-(CH₂)ₙ-NH₂ (n is an integer from 0 to 9) and H₂N-CH(COOH)-(CH₂)ₘ-Sₚ-(CH₂)_{q}-CH(COOH)-NH₂ (m, p and q are each independently integers from 0 to 9).

4. The drug according any one of claim 1 to claim 3, wherein the compound represented by formula (I) includes at least one selected from the group consisting of SMTP-0 below, SMTP-1 below, SMTP-4 below, SMTP-5D below, SMTP-6 below, SMTP-7 below, SMTP-8 below, SMTP-11 to 14 below, SMTP-18 to 29 below, SMTP-36 below, SMTP-37 below, SMTP-42 below, SMTP-43 below, SMTP-43D below, SMTP-44 below, SMTP-44D below, SMTP-46 below, and SMTP-47 below. 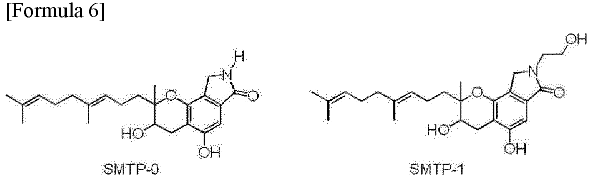 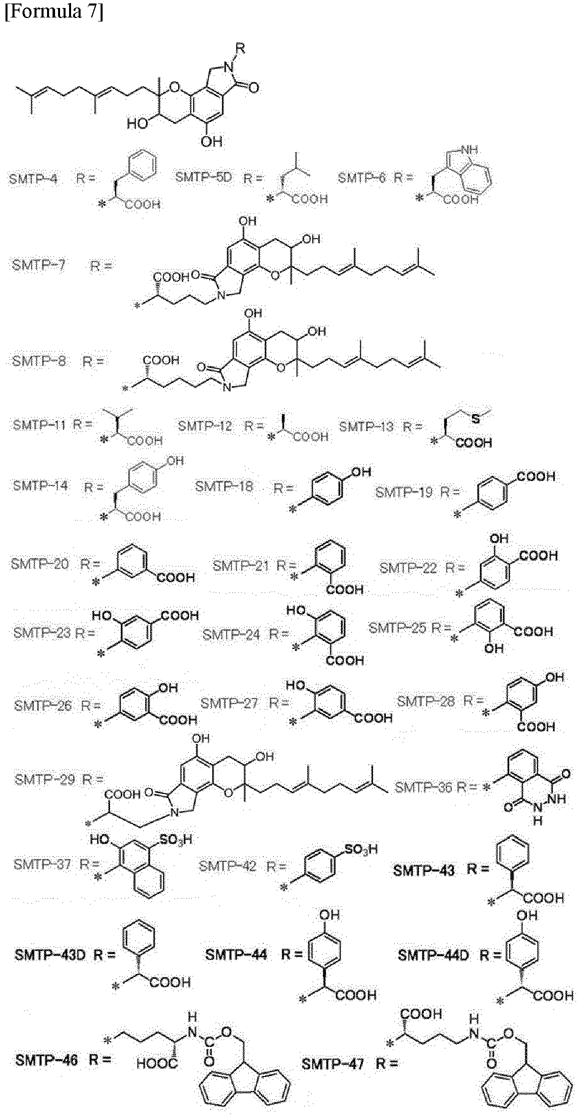 and in the formula, ^{∗} represents a binding site.

5. The drug according to claim 4, wherein the compound represented by formula (I) includes the SMTP-44D.

6. The drug according to claim 4, wherein the compound represented by formula (I) includes the SMTP-27.

7. The drug according to any one of claim 1 to claim 6, wherein the diabetes complication is diabetic neuropathy.

8. The drug according to claim 7, wherein the diabetic neuropathy is a polyneuropathy.

9. The drug according to claim 7, wherein the diabetic neuropathy is a mononeuropathy.

10. The drug according to any one of claim 7 to claim 9, wherein the diabetic neuropathy is neuropathy due to type 2 diabetes.

11. The drug according to any one of claim 1 to claim 6, wherein the diabetes complication is diabetic kidney disease.

12. The drug according to claim 11, wherein the diabetic kidney disease is diabetic nephropathy.

13. The drug according to claim 12, wherein the diabetic nephropathy is
a disease satisfying any one condition selected from the group consisting of:
eGFR value (mL/min/1.73 m²) being 30 or more and less than 90 and urinary albumin value (mg/gCr) being less than 30,
eGFR value (mL/min/1.73 m²) being 30 or more and less than 90 and urinary albumin value (mg/gCr) being 30 or more, and
eGFR value (mL/min/1.73 m²) being less than 30.

14. The drug according to any one of claim 11 to claim 13, wherein the diabetic kidney disease is nephropathy due to type 2 diabetes.

15. A method for treating or preventing a diabetes complication in a subject having a diabetes complication or at risk of developing a diabetes complication, comprising administering a dose of the drug according to any one of claim 1 to claim 14 which is effective for treating or preventing diabetes complications to the subject.

16. The method according to claim 15, wherein the dose effective for treating or preventing diabetes complications is 0.001 mg/kg of body weight to 200 mg/kg of body weight per dose to an adult.
